(19) Europäisches Patentamt
European Patent Office
Office européen des brevets

(11) Publication number : **0 123 904 B2**

# (12) NEW EUROPEAN PATENT SPECIFICATION

(45) Date of publication of the new patent specification : **03.02.93 Bulletin 93/05**

(51) Int. Cl.$^5$ : **G03C 1/494,** G03C 8/10

(21) Application number : **84103391.3**

(22) Date of filing : **27.03.84**

## (54) Heat developable photographic material.

The file contains technical information submitted after the application was filed and not included in this specification

(30) Priority : **29.03.83 JP 51655/83**

(43) Date of publication of application :
**07.11.84 Bulletin 84/45**

(45) Publication of the grant of the patent :
**24.06.87 Bulletin 87/26**

(45) Mention of the opposition decision :
**03.02.93 Bulletin 93/05**

(84) Designated Contracting States :
**DE FR GB**

(56) References cited :
**EP-A- 0 009 837**
**EP-A- 0 066 282**
**EP-A- 0 067 455**
**EP-A- 0 106 211**
**EP-A- 0 106 357**
**DE-A- 3 215 485**
**DE-A- 3 232 674**
**DE-B- 1 288 427**
**FR-A- 1 454 177**
**US-A- 3 241 967**
**US-A- 3 261 685**
**US-A- 3 312 550**

(73) Proprietor : **FUJI PHOTO FILM CO., LTD.**
**210 Nakanuma Minami Ashigara-shi**
**Kanagawa 250-01 (JP)**

(72) Inventor : **Nakamura, Koichi**
**c/o Fuji Photo Film Co., Ltd. No. 210,**
**Nakanuma**
**Minami Ashigara-shi Kanagawa (JP)**

(74) Representative : **Patentanwälte Grünecker, Kinkeldey, Stockmair & Partner**
**Maximilianstrasse 58**
**W-8000 München 22 (DE)**

EP 0 123 904 B2

## Description

The present invention relates to a heat-developable photographic material, which comprises a support having provided thereon a light sensitive silver halide, a binder, a dye-releasing redox compound which is capable of reducing the light-sensitive silver halide and which upon heating reacts with the light-sensitive silver halide to release a hydrophilic dye and a developing agent. Said heat-developable photographic material has improved developing properties.

Because of excellent photographic properties such as high speed and ease of tone control, silver halide photography has been used more extensively than any other photographic techniques such as electro-photography and diazo process. Conventionally, the formation of image in silver halide photography is effected by the wet process wherein the photographic material containing a silver halide is processed with a liquid developer. However, in recent years, various techniques of the dry process have been proposed, according to which the desired image can be formed more readily and rapidly than in the wet process.

Thermally developable photographic materials are known in the art and are shown, together with the methods for their processing, in "Fundamentals of Photographic Engineering", pp. 553-555, Corona Publishing Company, 1979: "Eizo Joho (Image Information)", p. 40, April 1978; and Neblets Handbook of Photography and Reprography, 7th ed., pp. 32-33, Van Nostrand Reinhold Company. Other pertinent references are U.S. Patents Nos. 3,152,904, 3,301,678, 3,392,020 and 3,457,075; British Patents Nos. 1,131,108 and 1,167,777; and Research Disclosure, June 1978, pp. 9-15 (RD-17029).

EP-A-106211 and EP-A-106357 disclose a heat-developable color photographic material comprising a support having thereon a light-sensitive silver halide, a hydrophilic binder and a dye releasing redox compound. As auxiliary developing agents pyrazolidones such as 1-phenyl-3-pyrazolidone or 1-phenyl-4-methyl-4-hydroxymethyl-1-phenyl-3-pyrazolidone are described.

Many methods have been proposed for producing color images by the dry process, and various methods are shown in the literature for providing a color image by the binding of a coupler to the oxidized product of a developing agent. U.S. Patent No. 3,531,286 shows the use of p-phenylenediamine reducing agents and phenolic or activated methylene couplers. U.S. Patent No. 3,761,270 shows the use of p-amino-phenolic reducing agents. Belgian Patent No. 802,519 and Research Disclosure, September 1975, pp. 31-32 shows the use of sulfonamide phenolic reducing agents. U.S. Patent No. 4,021,240 proposes the use of combinations of sulfonamide phenolic reducing agents and four-equivalent couplers.

However, according to these methods, both the image of reduced silver and a color image are formed in the exposed area after heat development, and this causes unavoidably staining of the color image. This problem can be solved either by removing the silver image by treatment with a liquid or by transferring the dye onto another layer, for example, an image-receiving layer on a sheet. However, it is difficult to transfer only the dye and leave the unreacted material in the exposed area.

A method wherein a nitrogen-containing heterocyclic group is introduced into the dye to form a silver salt and the dye is freed by heat development is shown in Research Disclosure, May 1978, pp. 54-58 (RD-16966). However, this method is not very practical since it is difficult to inhibit the dye release in the unexposed area and a sharp image is not easily obtained.

A method for producing a positive color image by bleaching heat-developable silver dye is shown in Research Disclosure, April 1976, pp. 30-32 (RD-14433), supra, December 1976, pp. 14-15 (RD-15227) and U.S. Patent No. 4,235,957. These references show useful dyes and specific methods of bleaching these dyes. However, this method requires the additional step of heating a superimposed activator sheet in order to accelerate the bleaching of the dye, and accordingly, an extra material is needed. Furthermore, the color image obtained is gradually reduced and bleached by the presence of free silver and hence is unable to withstand extended storage.

Formation of a color image with a leuco dye is shown in U.S. Patents Nos. 3,985,565 and 4,022,617. However, with this method, the leuco dye cannot be stably incorporated in the photographic material and the color image obtained is slowly discolored during storage.

We previously provided a new photographic material which was free from the defects of these conventional techniques and also provided an image forming process for that new material (Japanese Patent Application No. 157,758/81). This application proposed a photographic material capable of releasing a mobile hydrophilic dye by a simple step of heating in a substantially water-free state, as well as an image forming process characterized by transporting said mobile hydrophilic dye to a dye fixing layer primarily in the presence of a solvent.

As a result of further studies on the idea proposed in Japanese Patent Application No. 157,758/81, we have accomplished the present invention according to which the image of a mobile dye formed by heating in a substantially water-free state can be enhanced in density while reducing the undesired fog.

Therefore, the object of the present invention is to provide a heat-developable photographic material capable of forming a high-density color image by a short period of heat development effected either after or simultaneously with exposure and capable of being stored for an extended period before it is subjected to thermal processing.

Said object is achieved by a heat developable material which is characerized in that the developing agent is represented by the following general formula i

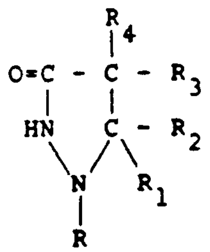

(i)

wherein R represents an unsubstituted or substituted aryl group and $R_1$, $R_2$, $R_3$ and $R_4$ which may be the same or different represent a hydrogen atom, an unsubstituted or substituted alkyl group, an unsubstituted or substituted aryl group, a carboxy group or an alkoxycarbonyl group with the exclusion of 1-phenyl-3-pyrazolidone and 1-phenyl-4-methyl-4-hydroxymethyl-3-pyrazolidone.

Examples of the aryl group having 6 to 22 carbon atoms designated by R includes phenyl, naphthyl, tolyl and xylyl groups. These aryl groups may be substituted by, for example, at least one substituent, such as a halogen atom (e.g., chlorine or bromine atom), an amino group, an alkoxy group having 1 to 22 carbon atoms, an aryloxy group having 6 to 22 carbon atoms, a hydroxy group, an aryl group having 6 to 22 carbon atoms, a carbonamide group, a sulfonamide group, an alkanoyloxy group having 1 to 22 carbon atoms, benzoyloxy group, ureido group, carbamate group, a carbamoyloxy group, a carbonate group, a carboxy group, and an alkyl group having 1 to 22 carbon atoms (e.g., methyl, ethyl or propyl). The substituent for the aryl group has up to 22 carbon atoms unless specified.

The alkyl groups designated by $R_1$, $R_2$, $R_3$ and $R_4$ are those having 1 to 10 carbon atoms (e.g., methyl, ethyl, propyl and butyl), and these alkyl groups may be substituted by a hydroxy group, an amino group having 1 to 22 carbon atoms, or a carbonyloxy group having 1 to 22 carbon atoms. An illustrative aryl group for $R_1$, $R_2$, $R_3$ and $R_4$ has 6 to 22 carbon atoms, such as phenyl, naphthyl, xylyl and tolyl groups. These aryl groups may be substituted by at least one halogen atom (e.g., chlorine or bromine), an alkyl group having 1 to 22 carbon atoms (e.g., methyl, ethyl or propyl), a hydroxy group and an alkoxy group having 1 to 22 carbon atoms (e.g., methoxy or ethoxy).

The carboxy group and the alkoxycarbonyl group represented by $R_1$, $R_2$, $R_3$ and $R_4$ have 1 to 22 carbon atoms and 2 to 23 carbon atoms. In the present invention, $R_1$, $R_2$, $R_3$ and $R_4$ are preferably a hydrogen atom, an alkyl group having 1 to 10 carbon atoms, a substituted alkyl group having 1 to 10 carbon atoms, and an aryl group having 6 to 22 carbon atoms. A hydrogen atom, a methyl group and a hydroxymethyl group are particularly preferred. Specific examples of the compounds of formula i are listed below.

i- 1

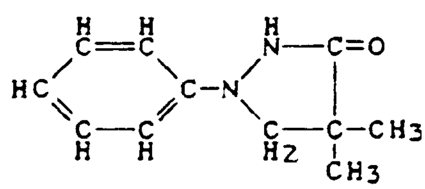

i- 2

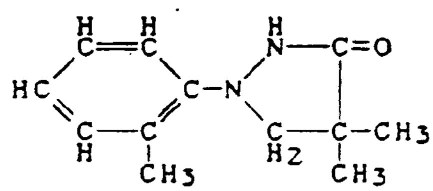

i- 3

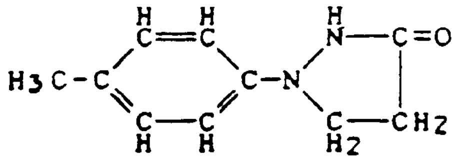

i- 4

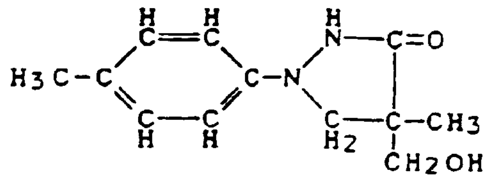

i- 5

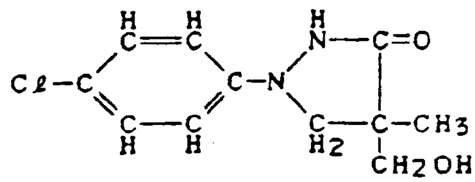

i- 6

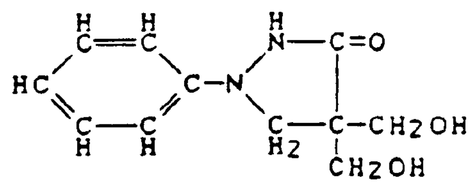

i- 7

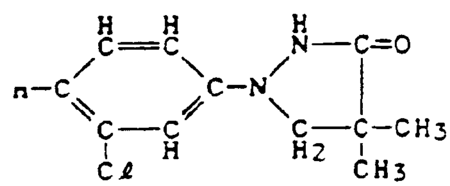

i- 8

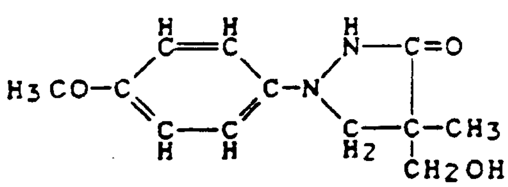

i- 9

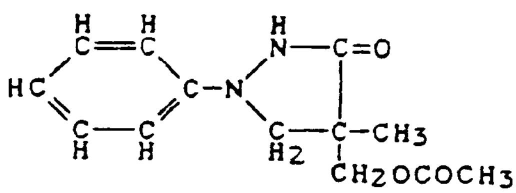

i- 10

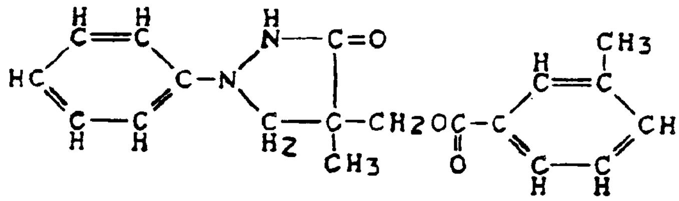

i- 11

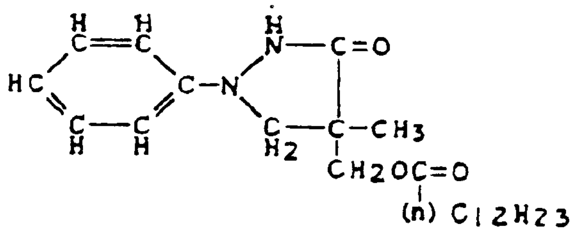

i- 12

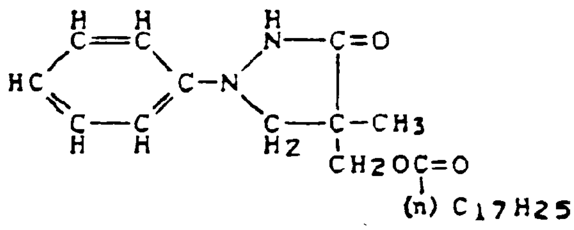

i- 13

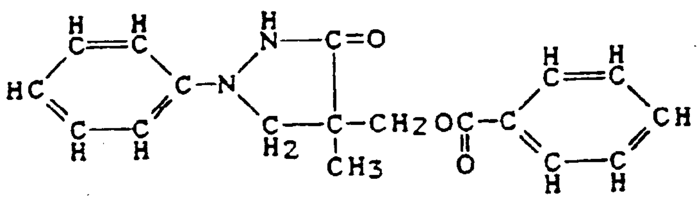

i- 14

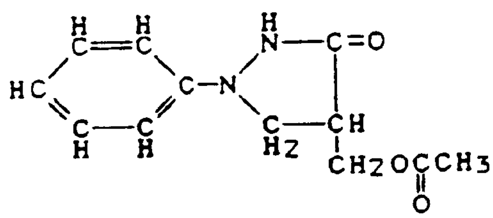

i- 15

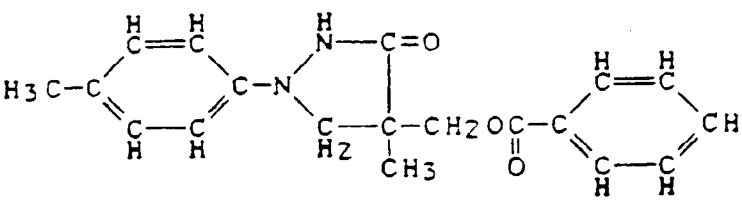

i- 16

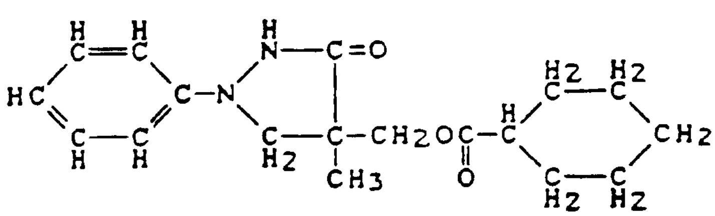

i- 17

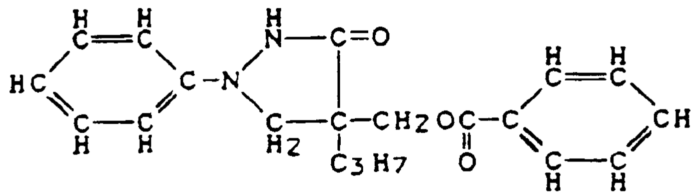

i- 18

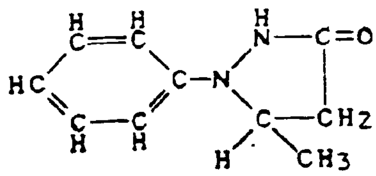

i- 19

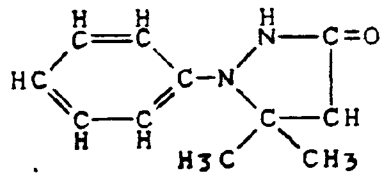

i-20

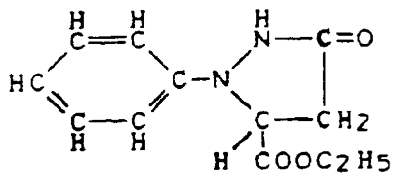

i- 21

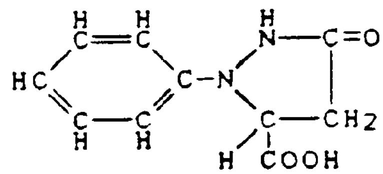

i- 22

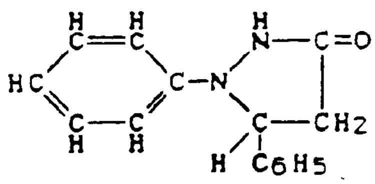

i-23

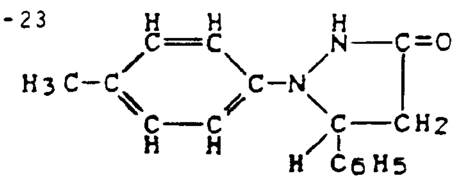

i- 24

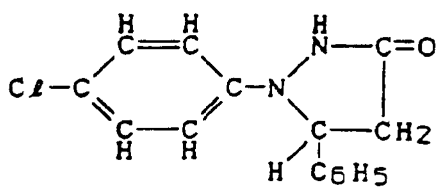

i- 25

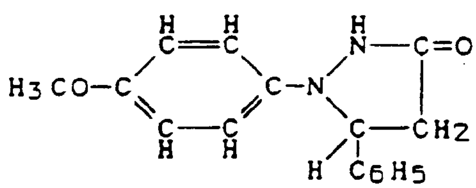

i- 26

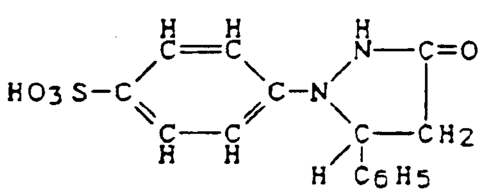

i-27

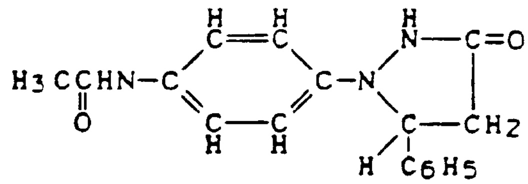

i-28

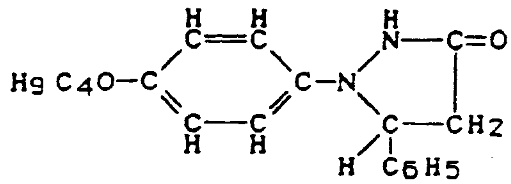

i-29

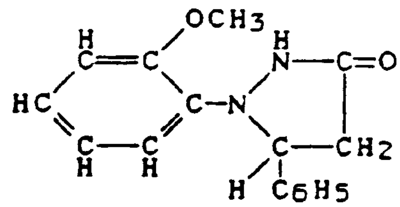

i-30

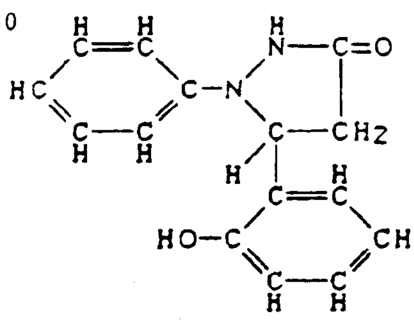

i-31

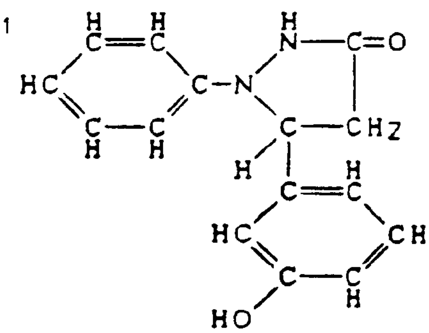

i-32

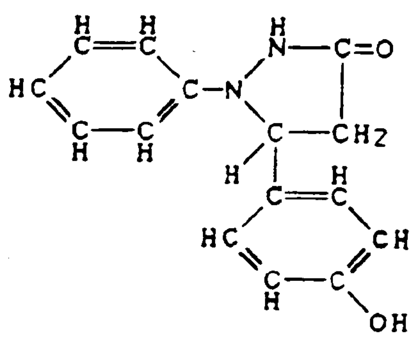

i-33

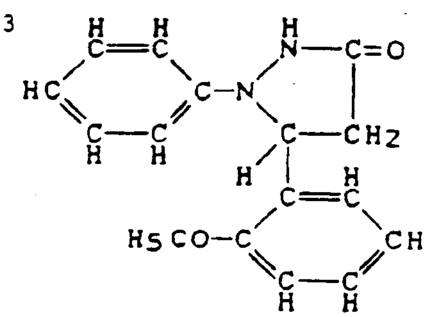

i-34

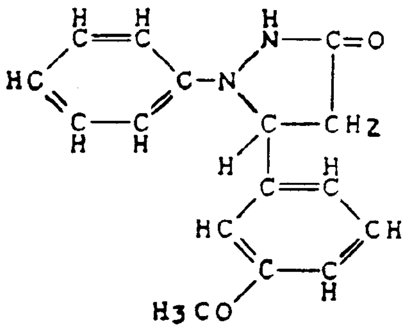

i-35

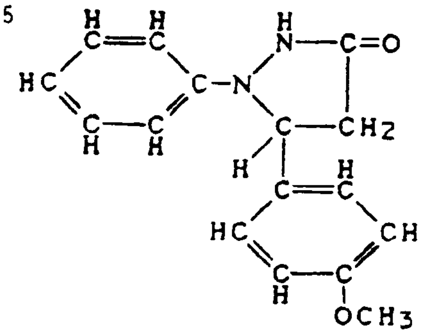

i-36

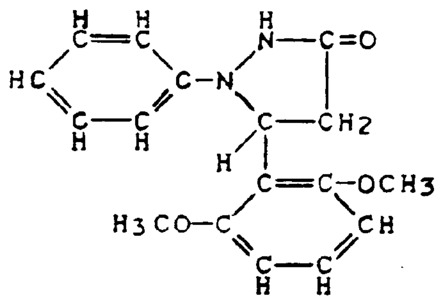

i-37

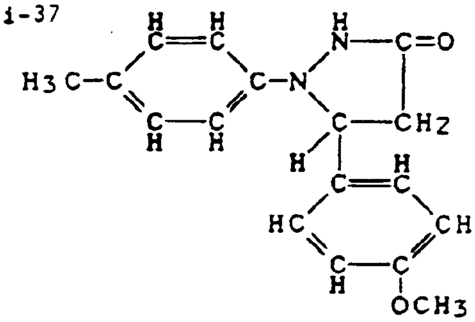

i-38

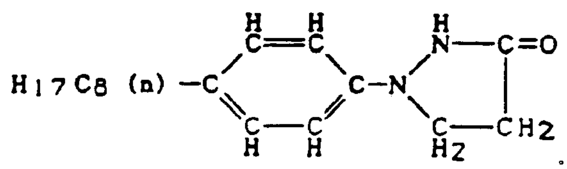

i-39

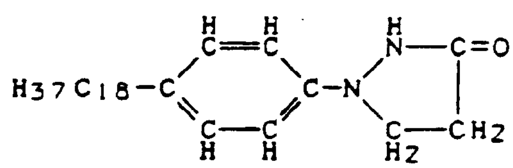

Among the typical compounds listed, those which are particularly preferred for the purposes of the present invention are I-12, I-13, I-14, I-15, I-17, I-18, I-19, I-24, I-25, I-26, I-27, I-30, I-31, and I-35 to I-41.

Synthesis of several compounds of formula i are shown below.

Synthesis 1 (compound i-12)

Acetonitrile (300 ml) was added to 4-methyl-4-methyl-1-phenyl-3-pyrazolidone (41.2 g) in a four-necked flask (1,000 ml capacity). While the outer jacket was cooled with cold water and the internal temperature held at 10°C, benzoyl chloride (31.0 g) was gradually added dropwise over a period of 30 minutes under stirring in a nitrogen stream. Thereafter, the outer jacket was removed, and the contents of the flask were stirred for 3 hours at room temperature and were left for 24 hours in a nitrogen atmosphere. After being concentrated to dryness, the reaction solution was dissolved in ethyl acetate and washed with 3% aqueous $NaHCO_3$. After washing with water, the ethyl acetate phase was dried over Glauber's salt and concentrated to dryness. The solids were crystallized with a mixed solvent of methanol and water and recrystallized with n-hexane/ethyl acetate. The yield of the product was 10.5 g. NMR, IR and elemental analyses showed that the product was the titled compound.

Synthesis 2 (compound i-25)

A hydrochloride salt of 4-methylphenylhydrazine (22.2 g), tert-butylhydroquinone (1.5 g), 28% sodium methoxide containing methanol (67.5 ml) and n-butanol (250 ml) were heated under stirring in a nitrogen stream. After distilling off methanol, ethyl cinnamate (29.6 g) was added dropwise over a period of 30 minutes. While n-butanol was distilled off at atmospheric pressure, the reaction solution was heated for 2 hours under stirring. After cooling, the reaction solution was neutralized with aqueous HCl, extracted with ethyl acetate, washed with water and dried over anhydrous magnesium sulfate. After distilling off the solvent, the dried product was crystallized with ethyl acetate to produce 7.7 g of the titled compound. m.p. 156-158°C.

Elemental analysis:

| | |
|---|---|
| Calcd. for $C_{16}H_{16}N_{20}$: | C 76.17, H 6.39, N 11.10(%) |
| Found: | C 76.20, H 6.19, N 11.07(%) |

Synthesis 3 (compound i-32)

A liquid mixture of phenylhydrazine (18 g), tert-butylhydroquinone (1.0 g), 28% sodium methoxide containing methanol (50.2 ml) and n-butanol (250 ml) was heated under stirring in a nitrogen stream. After distilling off methanol, ethyl n-hydroxycinnamate (32 g) in n-butanol (100 ml) was added dropwise over 30 minutes. While n-butanol was distilled off at atmospheric pressure, the reaction solution was heated for 3 hours under stirring. Thereafter, the reaction solution was cooled, neutralized with aqueous HCl, extracted with n-butanol and washed with saturated aqueous sodium chloride. The extract was dried over anhydrous magnesium sulfate. After distilling off the solvent, the residue was recrystallized with a mixed solvent of methanol and n-hexane (2:1) to obtain 14.1 g of the titled compound m.p. 188-189.5°C.

Elemental analysis:

| | |
|---|---|
| Calcd. for $C_{15}H_{14}N_2O_2$: | C 70.85, H 5.55, N 11.01(%) |
| Found: | C 70.84, H 5.43, N 11.17(%) |

Synthesis 4 (compound i-35)

A liquid mixture of phenylhydrazine (19.4 g), tert-butylhydroquinone (1.6 g), 28% sodium methoxide containing methanol (60.3 ml) and n-butanol (250 ml) was heated under stirring in a nitrogen stream. After distilling off methanol, ethyl n-methoxycinnamate (41.2 g) in n-butanol (100 ml) was added dropwise over 30 minutes. While distilling off n-butanol at atmospheric pressure, the reaction solution was heated under stirring for 1 hour. The reaction solution was then cooled and neutralized with aqueous HCl. The neutralized reaction solution was extracted with n-butanol, washed with saturated aqueous sodium chloride and dried over anhydrous magnesium sulfate. The solvent was distilled off and the resulting solids were recrystallized with methanol to obtain 30 g of the titled compound. m.p. 164 - 166°C.

Elemental analysis:

| | |
|---|---|
| Calcd. for $C_{16}H_{16}N_2O_2$: | C 71.62, H 6.01, N 10.44(%) |
| Found: | C 71.61, H 5.95, N 10.56(%) |

The compounds of formula i may be used over a certain range of concentrations. The useful concentration range is from 0.0005 mol to 20 mols per mol of silver, and a particularly useful range is from 0.001 to 4 mols per mol of silver.

The developing agent of the present invention may be introduced into the hydrophilic colloidal layer by various methods. One suitable method is shown in U.S. Patent No. 2,322,027 which uses a high-boiling organic solvent such as an alkyl ester of phthalic acid (e.g. dibutyl phthalate or dioctyl phthalate), a phosphate ester (e.g. diphenyl phosphate, triphenyl phosphate, tricresyl phosphate or dioctylbutyl phosphate), a citrate ester (e.g. tributyl acetylcitrate), a benzoate ester (e.g. octyl benzoate), an alkylamide (e.g. diethyllaurylamide), an aliphatic acid ester (e.g. dibutoxyethyl succinate or dioctyl azelate) or trimesic acid ester (e.g. tributyl trimesate). In another method, the developing agent is dissolved in an organic solvent having a boiling point of about 30-160°C, such as a lower alkyl acetate (e.g. ethyl acetate or butyl acetate), ethyl propionate, secondary butyl alcohol, methylisobutyl ketone, β-ethoxyethyl acetate, methyl cellosolve acetate or cyclohexanone, and the resulting solution is dispersed in the hydrophilic colloid. The high-boiling solvent may be used in admixture with the low-boiling solvent. The colloidal dispersion may also be formed with the aid of polymers as shown in Japanese Patent Publication No. 39853/76 and Japanese Patent Application (OPI) No. 59943/76 (the symbol OPI as used herein means an unexamined published Japanese Patent Application). According to other methods, the developing agent may be directly dispersed in an emulsion, or alternatively, the agent is first dissolved in water or an alcohol, and the resulting solution is dispersed in gelatin or an emulsion.

The developing agents of the present invention may be used in combination with themselves. They may be contained in one emulsion layer (blue-sensitive, green-sensitive or red-sensitive layer) or they may be contained in all of these emulsion layers. If desired, they may be incorporated in one or more layers adjacent to the emulsion layers (e.g. subbing layer, intermediate layer or protective layer).

The developing agents may be incorporated at any stage of the manufacture of the photographic material of the present invention, but generally, they may be incorporated immediately before the application of a coating solution.

In the photographic material of the present invention, by means of heating that is effected in a substantially water-free state either after or simultaneously with imagewise exposure, an oxidation-reduction reaction as catalyzed by the exposed photosensitive silver halide takes place between the photosensitive silver halide and the reducing dye-releasing redox compound. In consequence, not only the silver image but also the mobile hydrophilic dye image released from the dye-releasing redox compound that has been oxidized by the silver halide is formed in the exposed area. However, with the heat development alone, it often occurs that part of the dye-releasing redox compound remains unreacted in the exposed area and this is difficult to be distinguished from the released mobile hydrophilic dye image. In this case, the obtained color image which is made of a hydrophilic mobile dye can be transported to a dye fixing layer by placing said image in an atmosphere having affinity for the hydrophilic dye. Thereby, a color image of good quality that can be stored for an extended period can be produced. The atmosphere having affinity for the hydrophilic dye can be obtained either externally by supplying a solvent or internally by using a hydrophilic thermal solvent.

The above described theory of operation is essentially the same whether a negative emulsion or an autopositive emulsion is used in the photographic material of the present invention. With the use of an autopositive emulsion, a dye image having good color reproduction can be formed by the same processing as in the case of using a negative emulsion except that only the mobile dye image, but not the silver image, formed in the unexposed area is transported to the dye fixing layer.

In the present invention, the oxidation-reduction reaction between the photosensitive silver halide and the dye-releasing redox compound and the subsequent dye releasing reaction can be initiated by heating in a substantially water-free state. The term "heating" means the one effected to a temperature between 80 and 250°C, and the words "substantially water-free state" mean the reaction system which is in equilibrium with the moisture in air and which need not be supplied with water in order to initiate or accelerate the reaction. For the specific definition of this state, see "The Theory of the Photographic Process", 4th Ed., edited by T. H. James, Macmillan, p. 374.

According to the present invention, dyes to be released can be selected by properly selecting a dye-releasing redox compound, and hence, various colors can be reproduced. Therefore, the dye image obtained in the present invention includes not only a monochromatic image but also a multicolor image, and the monochromatic image may include the one formed by the mixing of two or more colors.

It has been generally understood that the dye releasing reaction is initiated by the attack of a nucleophilic reagent, and this reaction is normally carried out in an aqueous solution having a pH higher than 10. It is therefore quite unusual that with the photographic material of the present invention, a high degree of the dye releasing reaction can be attained by a simple means of heating in a substantially water-free state.

The two reactions described above proceed more smoothly in the presence of an organic silver salt as an oxidizing agent and a high image density can be obtained. Therefore, the presence of such an organic silver salt as oxidizing agent is a particularly preferred embodiment of the present invention.

The dye releasing redox compound which releases a hydrophilic diffusible dye used in the present invention is represented by the following general formula I:

$$Ra\text{-}SO_2\text{-}D \qquad (I)$$

wherein Ra represents a reducing group capable of being oxidized by the silver halide; and D represents an image forming dye portion containing a hydrophilic group.

Preferably the reducing group Ra in the dye releasing redox compound $Ra\text{-}SO_2\text{-}D$ has an oxidation-reduction potential to a saturated calomel electrode of 1.2 V or less measuring the polarographic half wave potential using acetonitrile as a solvent and sodium perchlorate as a base electrolyte. Preferred examples of the reducing group Ra include those represented by the following general formulae II to IX.

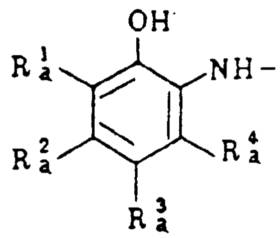

(II)

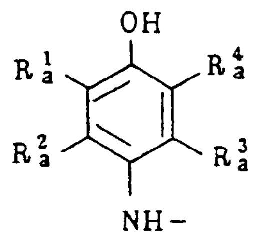

( III )

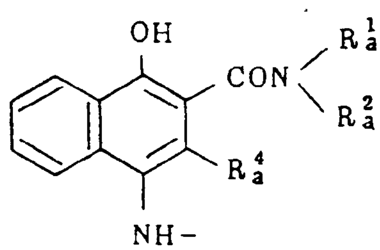

( IV )

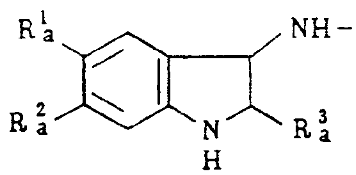

( V )

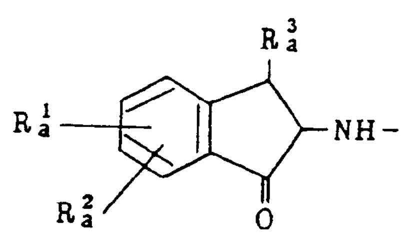

( VI )

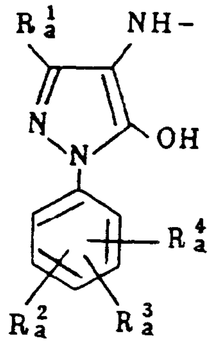

(VII)

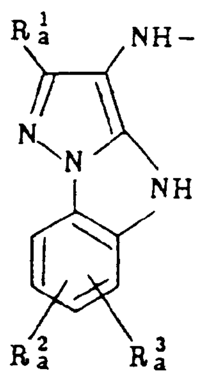

(VIII)

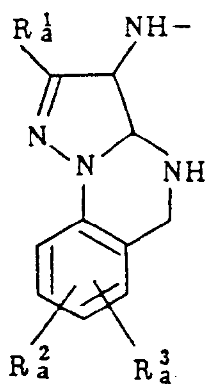

(IX)

wherein $R_a^1$, $R_a^2$, $R_a^3$ and $R_a^4$ each represents a hydrogen atom or a substituent selected from an alkyl group, a cycloalkyl group, an aryl group, an alkoxy group, an aryloxy group, an aralkyl group, an acyl group, an acylamino group, an alkylsulfonylamino group, an arylsulfonylamino group, an aryloxyalkyl group, an alkoxyalkyl group, an N-substituted carbamoyl group, an N-substituted sulfamoyl group, a halogen atom, an alkylthio group or an arylthio group. The alkyl moiety and the aryl moiety in the above described substituents may be further substituted with an alkoxy group, a halogen atom, a hydroxy group, a cyano group, an acyl group, an acylamino group, a substituted carbamoyl group, a substituted sulfamoyl group, an alkylsulfonylamino group, an arylsulfonylamino group, a substituted ureido group or a carboalkoxy group. Furthermore, the hydroxy group and the amino group included in the reducing group represented by Ra may be protected by a protective group capable of reproducing the hydroxy group and the amino group by the action of a nucleophilic agent.

In more preferred embodiments of the present invention, the reducing group Ra is represented by the following general formula X.

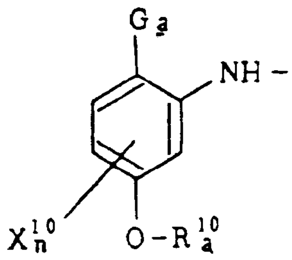

(X)

wherein Ga represents a hydroxy group or a group giving a hydroxy group upon hydrolysis; $R_a^{10}$ represents an alkyl group or an aromatic group; n represents an integer of 1 to 3; $X^{10}$ represents an electron donating substituent when n is 1 or substituents, which may be the same or different, one of the substituents being an electron donating group and the second or second and third substituents being selected from an electron donating group or a halogen atom when n is 2 or 3, respectively; wherein $X^{10}$ groups may form a condensed ring with each other or with $OR_a^{10}$; and the total number of the carbon atoms included in $R_a^{10}$ and $X^{10}$ is not less than 8.

Of the reducing groups represented by the general formula X more preferred reducing groups Ra are represented by the following general formulae Xa and Xb;

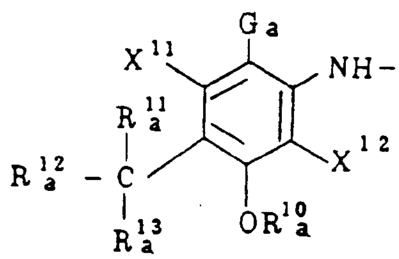

(Xa)

wherein Ga represents a hydroxy group or a group giving a hydroxy group upon hydrolysis; $R_a^{11}$ and $R_a^{12}$, which may be the same or different, each represents an alkyl group or $R_a^{11}$ and $R_a^{12}$ may be bonded to each other to form a ring; $R_a^{13}$ represents a hydrogen atom or an alkyl group; $R_a^{10}$ represents an alkyl group or an aromatic group; $X^{11}$ and $X^{12}$, which may be the same or different, each represents a hydrogen atom, an alkyl group, an alkoxy group, a halogen atom, an acylamino group or an alkylthio group; and $R_a^{10}$ and $X^{12}$ or $R_a^{10}$ and $R_a^{13}$ may be bonded to each other to form a ring,

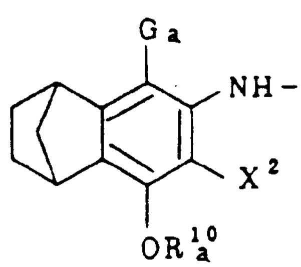

(Xb)

wherein Ga represents a hydroxy group or a group giving a hydroxy group upon hydrolysis; $R_a^{10}$ represents an alkyl group or an aromatic group; $X^2$ represents a hydrogen atom, an alkyl group, an alkoxy group, a halogen atom, an acylamino group or an alkylthio group; and $X^2$ and $R_a^{10}$ may be bonded to each other to form a ring.

Specific examples of the reducing groups represented by the above described general formulae X, Xa and Xb are described in U.S. Patent 4,055,428, Japanese Patent Application Nos. 12642/81 and 16130/81, respectively.

In other more preferred embodiments of the present invention, the reducing group Ra is represented by the following general formula XI

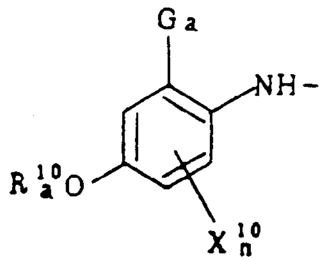

(XI)

wherein Ga; $X^{10}$, $R_a^{10}$ and n each has the same meaning as Ga, $X^{10}$, $R_a^{10}$ and n defined in the general formula X.

Of the reducing groups represented by the general formula XI, more preferred reducing groups Ra are represented by the following general formulae XIa, XIb and XIc

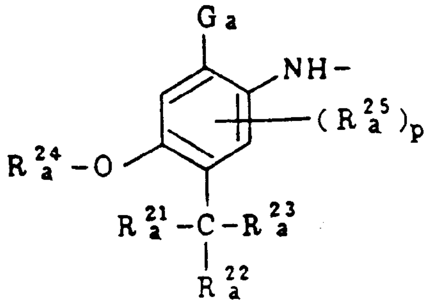

(XIa)

wherein Ga represents a hydroxy group or a group giving a hydroxy group upon hydrolysis; $R_a^{21}$ and $R_a^{22}$, which may be the same or different, each represents an alkyl group or an aromatic group, and $R_a^{21}$ and $R_a^{22}$ may be bonded to each other to form a ring; $R_a^{23}$ represents a hydrogen atom, an alkyl group or an aromatic group; $R_a^{24}$ represents an alkyl group or an aromatic group; $R_a^{25}$ represents an alkyl group, an alkoxy group, an alkylthio group, an arylthio group, a halogen atom or an acylamino group; p is 0, 1 or 2; $R_a^{24}$ and $R_a^{25}$ may be bonded to each other to form a condensed ring; $R_a^{21}$ and $R_a^{24}$ may be bonded to each other to form a condensed ring; $R_a^{21}$ and $R_a^{25}$ may be bonded to each other to form a condensed ring; and the total number of the carbon atoms included in $R_a^{21}$, $R_a^{22}$, $R_a^{23}$, $R_a^{24}$ and $(R_a^{25})_p$ is more than 7.

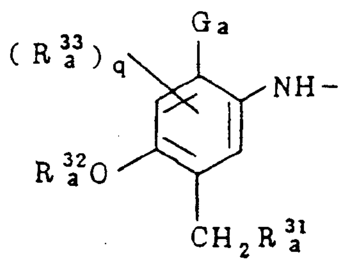

(XIb)

wherein Ga represents a hydroxy group or a group giving a hydroxy group upon hydrolysis; $R_a^{31}$ represents an alkyl group or an aromatic group; $R_a^{32}$ represents an alkyl group or an aromatic group; $R_a^{33}$ represents an alkyl group, an alkoxy group, an alkylthio group, an arylthio group, a halogen atom or an acylamino group; q is 0, 1 or 2; $R_a^{33}$ and $R_a^{33}$ may be bonded to each other to form a condensed ring; $R_a^{31}$ and $R_a^{32}$ may be bonded to each other to form a condensed ring; $R_a^{31}$ and $R_a^{33}$ may be bonded to each other to form a condensed ring; and the total number of the carbon atoms included in $R_a^{31}$, $R_a^{32}$ and $(R_a^{33})_q$ is more than 7.

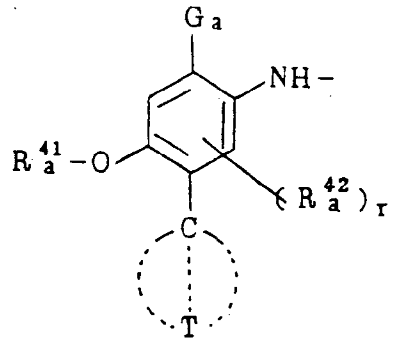

(XIc)

wherein Ga represents a hydroxy group or a group giving a hydroxy group upon hydrolysis; $R_a^{41}$ represents an alkyl group or an aromatic group; $R_a^{42}$ represents an alkyl group, an alkoxy group, an alkylthio group, an arylthio group, a halogen atom or an acylamino group; r is 0, 1 or 2; the group of

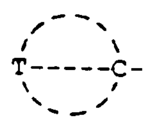

represents a group in which 2 to 4 saturated hydrocarbon rings are condensed, the carbon atom

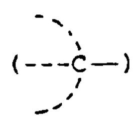

in the condensed ring which is connected to the phenol nucleus (or a precursor thereof), represents a tertiary carbon atom which composes one of the pivot of the condensed ring, a part of the carbon atoms (excluding the above described tertiary carbon atom) in the hydrocarbon ring may be substituted for oxygen atom(s), the hydrocarbon ring may have a substituent, and an aromatic ring may be further condensed to the hydrocarbon ring; $R_a^{41}$ or $R_a^{42}$ and the group of

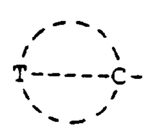

may be bonded to each other to form a condensed ring; and the total number of the carbon atoms included in $R_a^{41}$, ($R_a^{42}$) and the group of

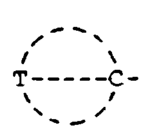

is not less than 7.

Specific examples of the reducing groups represented by the above described general formulae (XI), (XIa), (XIb) and (XIc) are described in Japanese Patent Application (OPI) Nos. 16131/81, 650/82 and 4043/82.

The essential part in the groups represented by the general formulae (III) and (IV) is a para(sulfonyl)aminophenol part. Specific examples of these reducing groups are described in U.S. Patents 3,928,312 and 4,076,529, U.S. Published Patent Application B 351,673, U.S. Patents 4,135,929 and 4,258,120. These groups are also effective for the reducing group Ra according to the present invention.

In still other more preferred embodiments of the present invention, the reducing group Ra is represented by the following general formula (XII).

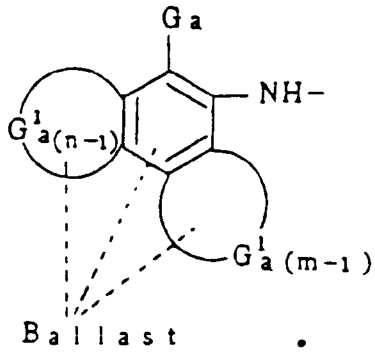

(XII)

wherein Ballast represents a diffusion-resistant group; Ga represents a hydroxy group or a precursor of a hydroxy group; $G^1_a$ represents an aromatic ring directly condensed to the benzene nucleus to form a naphthalene nucleus; and n and m are dissimilar positive integers of 1 to 2.

Specific examples of the reducing groups represented by the above described general formula XII are described in U.S. Patent 4,053,312.

The reducing groups represented by the above described general formulae V, VII, VIII and IX are characterized by containing a heterocyclic ring. Specific examples of the groups are described in U.S. Patent 4,198,235, Japanese Patent Application No. 46730/78 and U.S. Patent 4,273,855.

Specific examples of the reducing groups represented by the general formula VI are described in U.S. Patent 4, 149,892.

Characteristics required for the reducing group Ra are as follows.

1. It is rapidly oxidized by the silver halide to effectively release a diffusible dye for image formation by the function of the dye releasing activator.
2. The reducing group Ra has an extensive hydrophobic property, because it is necessary for the dye releasing redox compound to be diffusion-resistant in a hydrophilic or hydrophobic binder and that only the released dye has diffusibility.
3. It has excellent stability to heat and to the dye releasing activator and does not release the image forming dye until it is oxidized; and
4. It is easily synthesized.

In the following, specific examples of preferred reducing groups Ra which satisfy the above described requirements are shown. In the example, NH- represents the bond to the dye portion.

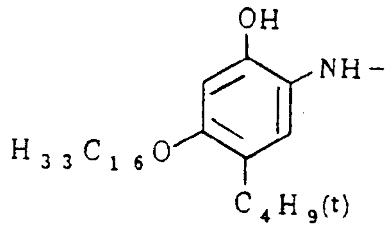

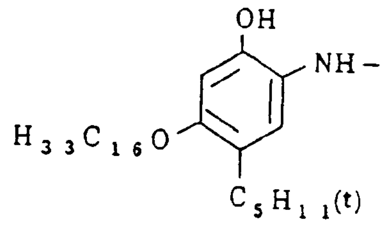

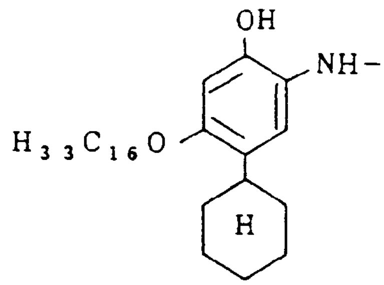

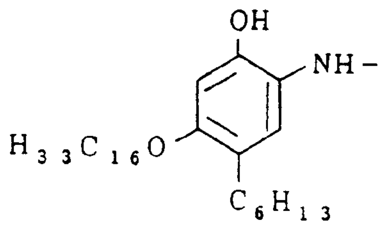

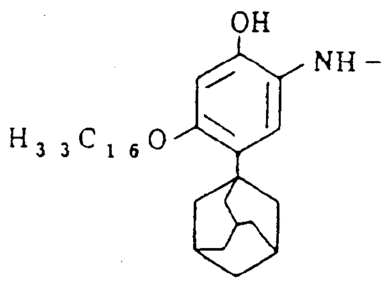

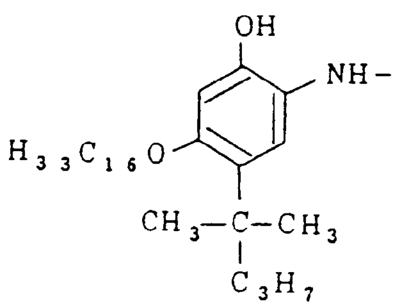

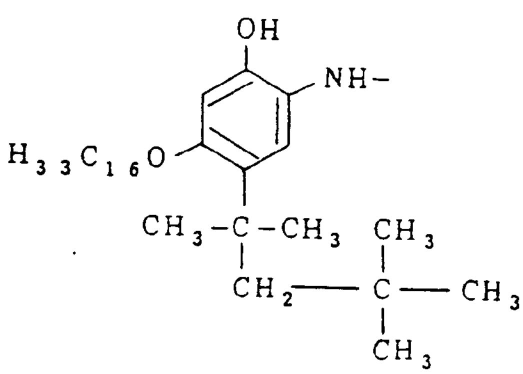

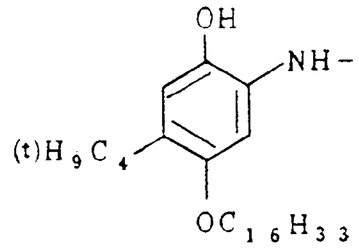

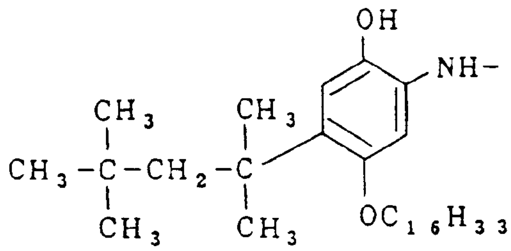

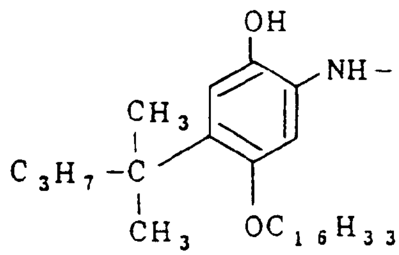

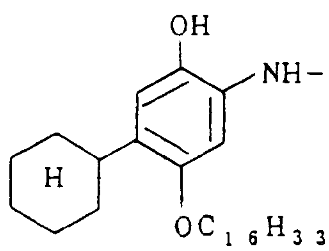

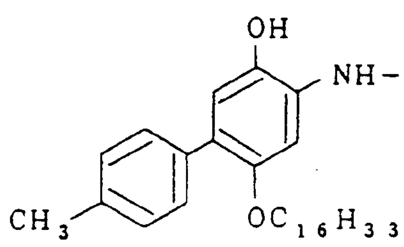

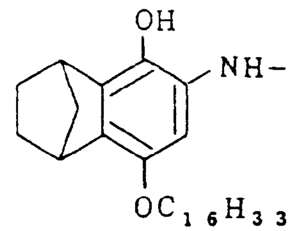

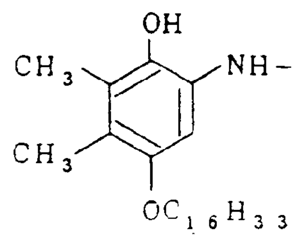

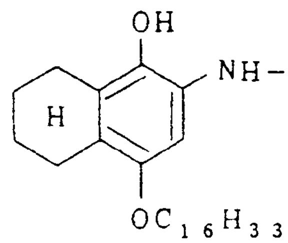

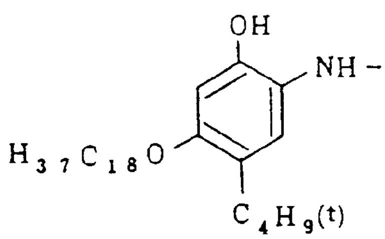

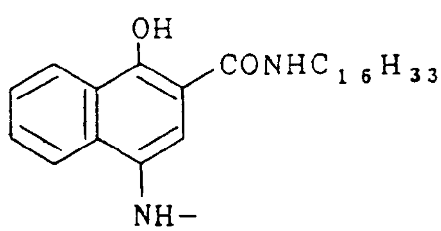

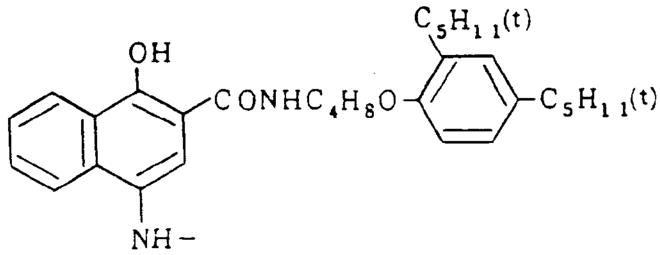

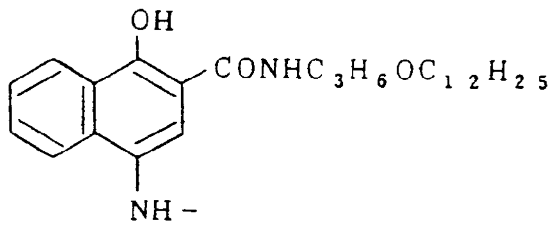

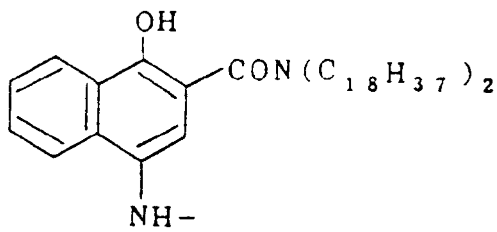

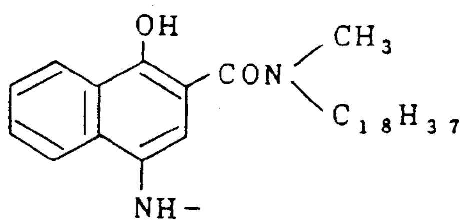

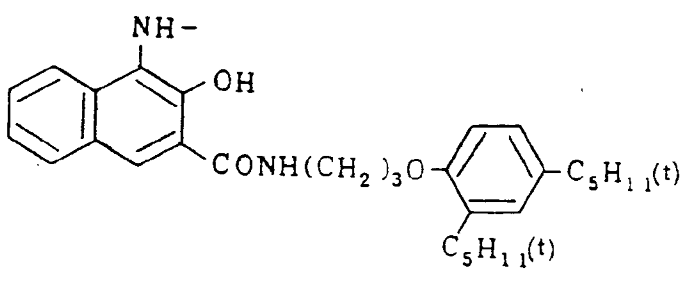

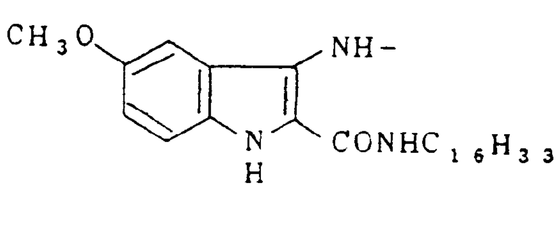

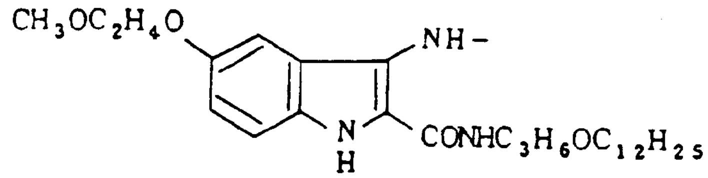

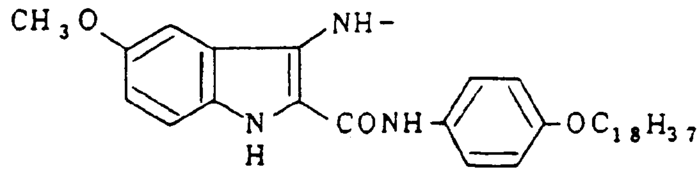

Examples of dyes which can be used for image forming dye include azo dyes, azomethine dyes, anthraquinone dyes, naphthoquinone dyes, styryl dyes, nitro dyes, quinoline dyes, carbonyl dyes and phthalocyanine dyes. Representative examples of them are set forth below and are classified by hue. Further, these dyes can be used in a form temporarily shifted to shorter wavelength region which is capable of regeneration during the development processing.

As the dye releasing redox compounds used in the present invention, the compounds as described, for example, in U.S. Patent 4,055,423, Japanese Patent Application (OPI) Nos. 12642/81, 16130/81, 16131/81, 650/82 and 4043/82, U.S. Patents 3,928,312 and 4,076,529, U.S. Published Patent Application B 351,673, U.S. Patents 4,135,929 and 4,198,235, Japanese Patent Application No. 46730/78, U.S. Patents 4,273,855, 4,149,892, 4,142,891 and 4,258,120, are also effective in addition to the above-described compounds.

Further, the dye releasing redox compounds which release a yellow dye as described, for example, in U.S. Patents 4,013,633, 4,156,609, 4,148,641, 4,164,987, 4,148,643, 4,183,755, 4,246,414, 4,268,625 and 4,245,023, Japanese Patent Application Nos. 71072/81, 25737/81, 138744/80, 134849/80, 106727/77 and 114930/76, can be effectively used in the present invention.

The dye releasing redox compounds which release a magenta dye as described, for example, in U.S. Patents 3,954,476, 3,932,380, 3,931,144, 3,932,381, 4,268,624 and 4,255,509, Japanese Patent Application Nos. 73057/81, 71060,81, 134850/80, 40402/80, 36804/80, 23628/78, 106727/77, 33142/80 and 53329/80, can be effectively used in the present invention.

The dye releasing redox compounds which release a cyan dye as described, for example, in U.S. Patents 3,929,760, 4,013,635, 3,942,987, 4,273,708, 4,148,642, 4,183,754, 4,147,544, 4,165,238, 4,246,414 and 4,268,625, Japanese Patent Application Nos. 71061/81, 47823/78, 8827/77 and 143323/78, can be effectively used in the present invention.

Two or more of the dye releasing redox compounds can be used together. In these cases, two or more dye releasing redox compounds may be used together in order to represent the same color or in order to represent black color.

The dye releasing redox compounds are suitably used in a range from 10 mg/$m^2$ to 15 g/$m^2$ and preferably in a range from 20 mg/$m^2$ to 10 g/$m^2$ in a total.

The process for preparing the dye releasing redox compound is described in European Patent Application (OPI) No. 76,492.

The dye releasing redox compound used in the present invention can be introduced into a layer of the light-sensitive material by known methods such as a method as described in U.S. Patent 2,322,027. In this case, an organic solvent having a high boiling point or an organic solvent having a low boiling point as described below can be used. For example, the dye releasing redox compound is dispersed in a hydrophilic colloid after being dissolved in an organic solvent having a high boiling point, for example, a phthalic acid alkyl ester (for example, dibutyl phthalate or dioctyl phthalate), a phosphoric acid ester (for example, diphenyl phosphate, triphenyl phosphate, tricresyl phosphate or dioctylbutyl phosphate) a citric acid ester (for example, tributyl acetylcitrate), a benzoic acid ester (for example, octyl benzoate), an alkylamide (for example, diethyl laurylamide), an aliphatic acid ester (for example, dibutoxyethyl succinate or dioctyl azelate) or a trimesic acid ester (for example, tributyl trimesate), or an organic solvent having a boiling point of about 30°C to 160°C, for example, a lower alkyl acetate such as ethyl acetate or butyl acetate, ethyl propionate, secondary butyl alcohol, methyl isobutyl ketone, β-ethoxyethyl acetate, methyl cellosolve acetate or cyclohexanone. The above-described organic solvents having a high boiling point and organic solvents having a low boiling point may be used as a

mixture thereof.

Further, it is possible to use a dispersion method using a polymer as described in Japanese Patent Publication No. 39853/76 and Japanese Patent Application No. 59943/76. Moreover, various surface active agents can be used when the dye releasing redox compound is dispersed in a hydrophilic colloid. For this purpose, the surface active agents illustrated in other parts of the specification can be used.

In the present invention, if necessary, a reducing agent may be used. The developing agent of the present invention is oxidized by the silver halide and/or the organic silver salt oxidizing agent to form its oxidized product having an ability to oxidize the reducing group Ra in the dye-releasing redox compound.

The following auxiliary developing agent may be used together with the developing agent of the present invention. The preferred auxiliary developing agent includes hydroquinone, alkyl substituted hydroquinones such as tertiary butylhydroquinone or 2,5-dimethylhydroquinone, catechols, pyrogallols, halogen substituted hydroquinones such as chlorohydroquinone or dichlorohydroquinone, alkoxy substituted hydroquinones such as methoxyhydroquinone, and polhydroxybenzene derivatives such as methyl hydroxynaphthalene. Further, methyl gallate, ascorbic acid, ascorbic acid derivatives, hydroxylamines such as N,N-di(2-ethoxyethyl)hydroxylamine, pyrazolidones such as 1-phenyl-3-pyrazolidone, 4-methyl-4-hydroxymethyl-1-phenyl-3-pyrazolidone, reductones and hydroxy tetronic acids are useful.

The auxiliary developing agent can be used in an amount of a fixed range. A suitable range is 0.0005 time by mol to 20 times by mol based on silver. A particularly suitable range is 0.001 time by mole to 4 times by mol.

The silver halide used in the present invention includes for example silver chloride, silver chlorobromide, silver chloroiodide, silver bromide, silver iodobromide, silver chloroiodobromide and silver iodide.

In the embodiment of the present invention in which the organic silver salt oxidizing agent is not used together with but the silver halide is used alone, particularly preferred silver halide is silver halide partially containing a silver iodide crystal in its grain. That is, the silver halide which shows the X-ray diffraction pattern of pure silver iodide is particularly preferred.

In photographic materials a silver halide containing two or more kinds of halogen atoms can be used. Such a silver halide is present in the form of a completely mixed crystal in -a conventional silver halide emulsion. For.example, the grain of silver iodobromide shows X-ray diffraction pattern at a position corresponding to the mixed ratio of silver iodide crystal and silver bromide crystal but not at a position corresponding to pure silver iodide crystal and pure silver bromide crystal separately.

Particularly preferred examples of silver halide used in the present invention include silver chloroiodide, silver iodobromide, and silver chloroiodobromide each containing silver iodide crystals in its grain and showing X-ray diffraction pattern of silver iodide crystal.

The process for preparing those silver halides is explained by means of silver iodobromide.

The silver iodobromide is prepared by first adding silver nitrate solution to potassium bromide solution to form silver bromide particles and then adding potassium iodide to the mixture.

Two or more kinds of silver halides in which a particle size and/or a halogen composition are different from each other may be used in mixture.

An average particle size of the silver halide used in the present invention is preferably from 0.001 μm to 10 μm and more preferably from 0.001 μm to 5 μm.

The silver halide used in the present invention may be used as it is. However, it may be chemically sensitized with a chemical sensitizing agent such as compounds of sulfur, selenium or tellurium, or compounds of gold, platinum, palladium, rhodium or iridium, a reducing agent such as tin halide, or a combination thereof. The details thereof are described in T.H. James, The Theory of the Photographic Process, the Fourth Edition, Chapter 5, pages 149 to 169.

In a particularly preferred embodiment of the present invention, an organic silver salt oxidizing agent is used. The organic silver salt oxidizing agent is a silver salt which forms a silver image by reacting with the above-described image forming substance or a reducing agent coexisting, if necessary, with the image forming substance, when it is heated to a temperature of above 80°C and, preferably, above 100°C in the presence of exposed silver halide. By the existence of the organic silver salt osidizing agent, the light-sensitive material which provides higher color density can be obtained.

The silver halide used in this case does not always necessarily have to have the characteristic that the silver halide contains pure silver iodide crystals in the case of using the silver halide alone. Any silver halide which is known in the art can be used.

Examples of such organic silver salt oxidizing agents include those described in European Patent Application No. 76,492.

A silver salt of an organic compound having a carboxy group can be used. Typical examples thereof include a silver salt of an aliphatic carboxylic acid and a silver salt of an aromatic carboxylic acid.

In addition, a silver salt of a compound containing a mercapto group or a thione group and a derivative thereof can be used.

Further, a silver salt of a compound containing an imino group can be used. Examples of these compounds include a silver salt of benzotriazole and a derivative thereof as described in Japanese Patent Publication Nos. 30270/69 and 18416/70, for example, a silver salt of benzotriazole, a silver salt of alkyl substituted benzotriazole such as a silver salt of methylbenzotriazole,

a silver salt of a halogen substituted benzotriazole such as a silver salt of 5-chlorobenzotriazole,

a silver salt of carboimidobenzotriazole such as a silver salt of butylcarboimidobenzotriazole, a silver salt of 1,2,4-triazole or 1-H-tetrazole as described in U.S. Patent 4,220,709, a silver salt of carbazole, a silver salt of saccharin, a silver salt of imidazole and ain imidazole derivative.

Moreover, a silver salt as described in Research Disclosure, Vol. 170, No, 17029 (June, 1978) and an organic metal salt such as copper stearate, are the organic metal salt oxidizing agent capable of being used in the present invention.

Methods of preparing these silver halide and organic silver salt oxidizing agents and manners of blending them are described in Research Disclosure, No. 17029, Japanese Patent Application Nos. 32928/75 and 42529/76, U.S. Patent 3,700,458, and Japanese Patent Application Nos. 13224/74 and 17216/75.

A suitable coating amount of the light-sensitive silver halide and the organic silver salt oxidizing agent employed in the present invention is in total from 50 $mg/m^2$ to 10 $g/m^2$ calculated as an amount of silver.

The dye releasing reaction is accelerated by the presence of a dye releasing activator as a nucleophilic reagent. If the organic silver salt oxidizing agent is used as the oxidizing agent, the silver halide must be present in an effective distance from the organic silver salt in order to ensure the early initiation of the dye releasing reaction, and therefore, the silver halide and the organic silver salt are preferably present within the same layer.

Unlike the development in wet process, heat development is limited with respect to the diffusion of reactive molecular species and hence requires a prolonged reaction time. However, if too much time is spent in the heating for development, thermal reaction in the unexposed area proceeds to such an extent that an undesired fog is produced.

In the present invention, it is possible to use a thermal solvent. The term "thermal solvent" means a non-hydrolyzable organic material which melts at a temperature of heat treatment and melts at a lower temperature of heat treatment when it is present together with other components. Preferred examples of thermal solvents include compounds which can act as a solvent for the developing agent and compounds having a high dielectric constant which accelerate physical development of silver salts. Examples of preferred thermal solvents include those described in European Patent Application No. 76,492.

Heat-development under a presence of the thermal solvent accelerates developing rate and improves image quality. Although the role of the thermal solvent is not entirely clear, it is believed that the thermal solvent supports the diffusion of reactive molecules.

The light-sensitive silver halide and the organic silver salt oxidizing agent used in the present invention are prepared in the binder as described below. Further, the dye releasing redox compound and developing agent are dispersed in the binder described below.

The binder which can be used in the present invention can be employed individually or in a combination thereof. A hydrophilic binder is used as the binder according to the present invention. The typical hydrophilic binder is a transparent or translucent hydrophilic colloid, examples of which include a natural substance, for example, protein such as gelatin, or a gelatin derivative, a cellulose derivative, a polysaccharide such as starch or gum arabic, and a synthetic polymer, for example, a water-soluble polyvinyl compound such as polyvinyl alcohol, polyvinyl pyrrolidone acrylamide polymer.

The silver halide used in the present invention can be spectrally sensitized with methine dyes or other dyes. Suitable dyes which can be employed include cyanine dyes, merocyanine dyes, complex cyanine dyes, complex merocyanine dyes, holopolar cyanine dyes, hemicyanine dyes, styryl dyes, and hemioxonol dyes. Of these dyes, cyanine dyes, merocyanine dyes and complex merocyanine dyes are particularly useful. Any conventionally utilized nucleus for cyanine dyes, such as basic heterocyclic nuclei, can be contained in these dyes, for example a pyrroline nucleus, an oxazoline nucleus, a thiazoline nucleus, a pyrrole nucleus, an oxazole nucleus, a thiazole nucleus, a selenazole nucleus, an imidazole nucleus, a tetrazole nucleus or a pyrridine nucleus, and further, nuclei formed by condensing alicyclic hydrocarbon rings with these nuclei and nuclei formed by condensing aromatic hydrocarbon rings with these nuclei, for example, an indolenine nucleus, a benzindolenine nucleus, an indole nucleus, a benzoxazole nucleus, a naphthoxazole nucleus, a benzothiazole nucleus, a naphthothiazole nucleus, a benzoselenazole nucleus, a benzimidazole nucleus, or a quinoline nucleus, are appropriate. The carbon atoms of these nuclei may also be substituted.

As nuclei having a ketomethylene structure, 5- or 6-membered heterocyclic nuclei such as a pyrazolin-5-one nucleus, a thiohydantoin nucleus, a 2-thiooxazolidin-2,4-dione nucleus, a thiazolidin-2,4-dione nucleus, a

rhodanine nucleus or a thiobarbituric acid nucleus, may also be used in merocyanine dyes and complex merocyanine dyes.

These sensitizing dyes can be employed individually, and can also be employed in combination thereof. A combination of sensitizing dyes is often used, particularly for the purpose of supersensitization. Representative examples thereof are described in U.S. Patents 2,688,545, 2,977,229, 3,397,060, 3,522,052, 3,527,641, 3,617,293, 3,628,964, 3,666,480, 3,672,898, 3,679,428, 3,703,377, 3,769,301, 3,814,609, 3,837,862 and 4,026,707, British Patents 1,344,281 and 1,507,803, Japanese Patent Publication Nos. 4936/68 and 12375/78, Japanese Patent Application Nos. 110618/77 and 109925/77.

The sensitizing dyes may be present in the emulsion together with dyes which themselves do not give rise to spectrally sensitizing effects but exhibit a supersensitizing effect or materials which do not substantially absorb visible light but exhibit a supersensitizing effect. For example, aminostilbene compounds substituted with a nitrogen-containing heterocyclic group (e.g., those described in U.S. Patents 2,933,390 and 3,635,721 ), aromatic organic acid-formaldehyde condensates (e.g., those described in U.S. Patent 3,743,510), cadmium salts or azaindene compounds, can be present. The combinations described in U.S. Patents, 3,615,613, 3,615,641, 3,617,295 and 3,635,721 are particularly useful.

In the present invention, various kinds of dye releasing activators can be used. The dye releasing activator means a substance which accelerates the oxidation-reduction reaction between the light-sensitive silver halide and/or the organic silver salt oxidizing agent and dye releasing redox compound, or accelerates release of a dye by means of its nucleophilic action to the oxidized dye releasing redox compound in the dye releasing reaction subsequently occurred, and a base and a base precursor can be used. It is particularly advantageous to use these dye releasing activators in order to accelerate the reactions in the present invention.

Examples of preferred bases are amines which include trialkylamines, hydroxylamines, aliphatic polyamines, N-alkyl substituted aromatic amines, N-hydroxyalkyl substituted aromatic amines and bis[p-(dialkylamino )phenyl]methanes. Further, betaine tetramethylammonium iodide and diaminobutane dihydrochloride as described in U.S. Patent 2,410,644, and urea and organic compounds including amino acids such as 6-aminocaproic acid as described in U.S. Patent 3,506,444 are useful. The base precursor is a substance which releases a basic component by heating. Examples of typical base precursors are described in British Patent 998,949. A preferred base precursor is a salt of a carboxylic acid and an organic base, and examples of the suitable carboxylic acids include trichloroacetic acid and trifluoroacetic acid and examples of the suitable bases include guanidine, piperidine, morpholine, p-toluidine and 2-picoline. Guanidine trichloroacetate as described in U.S. Patent 3,220,846 is particularly preferred. Further, aldonic amides as described in Japanese Patent Application No. 22625/75 are preferably used because they decompose at a high temperature to form bases.

These dye releasing activators can be used in a broad range. A useful range is up to 50% by weight based on the amount of a dry layer coated of the light-sensitive material. A range of 0.01 % by weight to 40% by weight is more preferred.

The dye releasing activators may be incorporated in any layer of the photographic material or in any layer of the dye fixing material.

It is advantageous to use a compound represented by the general formula described below in the heat-developable color photographic material in order to accelerate development and accelerate release of a dye.

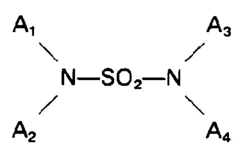

(A)

wherein $A_1$, $A_2$, $A_3$ and $A_4$, which may be the same or different, each represents a hydrogen atom or a substituent selected from an alkyl group, a substituted alkyl group, a cycloalkyl group, an aralkyl group, an aryl group, a substituted aryl group and a heterocyclic group; and $A_1$ and $A_2$ or $A_3$ and $A_4$ may combine with each other to form a ring.

The above-described compound can be used in a broad range. A useful range is up to 20% by weight based on the amount of a dry layer coated of the light-sensitive material. A range of 0.1 % by weight to 15% by weight is more preferred.

It is advantageous to use a water releasing compound in the present invention in order to accelerate the dye releasing reaction.

A water releasing compound means a compound which releases water by decomposition during heat development. These compounds are particularly known in the field oif printing of fabrics, and $NH_4Fe(SO_4)_2 \cdot 12H_2O$, as described in Japanese Patent Application No. 88386/75 is useful.

A support used in the light-sensitive material or used as the dye fixing material, if desired, according to the present invention is a material which can endure the processing temperature. As an ordinary support, not only glass, paper, metal or analogues thereto may be used, but also an acetyl cellulose film, a cellulose ester film, a polyvinyl acetal film, a polystyrene film, a polycarbonate film, a polyethylene terephthalate film, and a film related thereto or a plastic material may be used. Further, a papter support laminated with a polymer such as polyethylene, can be used. The polyesters described in U.S. Patents 3,634,089 and 3,725,070 are preferably used. The most preferred support is polyethylene terephthalate film.

The photographic emulsion layer and other hydrophilic colloid layers in the light-sensitive material of the present invention may contain various surface active agents for various purposes, for example, as coating aids, or for prevention of electrically charging, improvement of lubricating property, emulsification, prevention of adhesion or improvement of photographic properties (for example, acceleration of development, rendering hard tone or sensitization).

For example, it is possible to use nonionic surface active agents such as saponin (steroid saponin), alkylene oxide derivatives (for example, polyethylene glycol, polyethylene glycol/polypropylene glycol condensates, polyethylene glycol alkyl ethers or polyethylene glycol alkylaryl ethers, polyethylene glycol esters, polyethylene glycol sorbitan esters, polyalkylene glycol alkylamines or amides or polyethylene oxide adducts of silicone), glycidol derivatives (for example, alkenylsuccinic acid polyglycerides or alkylphenol polyglycerides), polyhydric alcohol aliphatic acid esters or saccharide alkyl esters, anionic surface active agents containing acid groups such as a carboxy group, a sulfo group, a phospho group, a sulfate group or a phosphate group, such as alkylcarboxylic acid salts, alkylsulfonate salts, alkylbenzenesulfonate salts, alkylnaphthalenesulfonate salts, alkyl sulfuric acid esters, alkylphosphoric acid ester, N-acyl-N-alkyltaurines, sulfosuccinic acid esters, sulfoalkyl polyoxyethylene alkylphenyl ethers or polyoxyethylene alkylphosphoric acid esters; ampholytic surface active agents such as amino acids, aminoalkylsulfonic acids, aminoalkylsulfuric acid esters or phosphoric acid esters, alkylbetaines or amine oxides and cationic surface active agents such as alkylamine salts, aliphatic or aromatic quaternary ammonium salts, heterocyclic quaternary ammonium salts such as pyridinium salts or imidazolium salts aliphatic or heterocyclic phosphonium salts or aliphatic or heterocyclic sulfonium salts.

Of the above-described surface active agents, polyethylene glycol type nonionic surface active agents having a recurring unit of ethylene oxide in the molecules may be preferably incorporated into the light-sensitive material. It is particularly preferred that the molecule contains 5 or more of the recurring units of ethylene oxide.

The nonionic surface active agents capable of satisfying the above-described conditions are well known as to their structures, properties and methods of synthesis. These nonionic surface active agents are widely used even outside this field. Representative references relating to these agents include: Surfactant Science Series, Vol. 1, Nonionic Surfactants (edited by Martin J. Schick, Marcel Dekker Inc., 1967), and Surface Active Ethylene Oxide Adducts (edited by Schoufeldt N. Pergamon Press, 1969). Among the nonionic surface active agents described in the above-mentioned references, those capable of satisfying the above-described conditions are preferably employed in connection with the present invention.

The nonionic surface active agents can be used individually or as a mixture of two or more of them.

The polyethylene glycol type nonionic surface active agents can be used in an amount of less than 100% by weight, preferably less than 50% by weight, based on a hydrophilic binder.

The light-sensitive material of the present invention may contain a cationic compound containing a pyridinium salt. Examples of the cationic compounds containing a pyridinium group used are described in PSA Journal Section B 36 (1953), U.S. Patents 2,648,604 and 3,671,247, Japanese Patent Publication Nos. 30074/69 and 9503/69.

Further, in the present invention, it is possible to use a compound which activates development and stabilizes the image at the same time. Particularly, it is preferred to use isothiuroniums including 2-hydroxyethylisothiuronium trichloroacetate as described in U.S. Patent 3,301,678, bisisothiuroniums including 1,8-(3,6-dioxaoctane)bis(isothiuronium trifluoroacetate), as described in U.S. Patent 3,669,670, thiol compounds as described in German Patent Application No. 2,162,714, thiazolium compounds such as 2-amino-2-thiazolium trichloroacetate or 2-amino-5-bromoethyl-2-thiazolium trichloroacetate, described in U.S. Patent 4,012,260, compounds having a-sulfonylacetate as an acid part such as bis(2-amino-2-thiazolium)methylene-bis(sulfonylacetate) or 2-amino-2-thiazolium phenylsulfonylacetate, as described in U.S. Patent 4,060,420, and compounds having 2-carboxycarboxamide as an acid part as described in U.S. Patent 4,088,496.

In the present invention, though it is not always necessary to further incorporate substances or dyes for preventing irradiation or halation in the light-sensitive material, becuase the light-sensitive material is colored by the dye releasing redox compound, it is possible to add filter dyes or light absorbing materials into the light-sensitive material, as described in Japanese Patent publication No. 3692/73 and U.S. Patents 3,253,921, 2,527,583 and 2,956,879, in order to further improve sharpness. It is preferred that these dyes have a thermal bleaching property. For example, dyes as described in U.S. Patents 3,769,019, 3,745,009 and 3,615,432 are

preferred.

The light-sensitive material used in the present invention may contain, if necessary, various additives known for the heat-developable light-sensitive materials and may have a layer other than the light-sensitive layer, for example, an antistatic layer, an electrically conductive layer, a protective layer, an intermediate layer, an antihalation layer or a strippable layer.

Examples of various additives include those described in Research Disclosure, Vol. 170, No. 17029 (June, 1978), for example, plasticizers, dyes for improving sharpness, antihalation dyes, sensitizing dyes, matting agents, fluorescent whitening agents and fading preventing agents.

If necessary, two or more layers may be coated at the same time by the method as described in U.S. Patent 2,761,791 and British Patent 837,095.

Various means for exposure can be used in the present invention. Latent images are obtained by imagewise exposure by radiant rays including visible rays. Generally, light sources used in this invention include tungsten lamps, mercury lamps, halogen lamps such as iodine lamps, xenon lamps, laser light sources, CRT light sources, fluorescent tubes and light-emitting diodes.

In the present invention, after the heat-developable color photographic material is exposed to light, the resulting latent image can be developed by heating the whole material to a suitably elevated temperature, for example, about 80°C to about 250°C for about 0.5 second to about 300 seconds. A high temperature or lower temperature can be utilized to prolong or shorten the heating time, if it is within the above-described temperature range. Particularly, a temperature range of about 110°C to about 160°C is useful.

As the heating means, a simple heat plate, iron, heat roller, heat generator utilizing carbon or titanium white, or analogues thereof may be used.

In the present invention, a specific method for forming a color image by heat development comprises transfer of a hydrophilic mobile dye. For this purpose, the heat-developable color photographic material of the present invention is composed of a support having thereon a light-sensitive layer containing at least silver halide, if necessary, an organic silver salt oxidizing agent, a dye releasing redox compound which is also a reducing agent for the organic silver salt oxidizing agent, a developing agent and a binder, and a dye fixing layer capable of receiving the hydrophilic diffusible dye formed in the light-sensitive layer.

The above-described light-sensitive layer and the dye fixing layer may be formed on the same support, or they may be formed on different supports, respectively. The dye fixing layer can be stripped off the light-sensitive layer. For example, after the heat-developable color photographic material is exposed imagewise to light, it is developed by heating uniformly and thereafter the dye fixing layer or the light-sensitive layer is peeled apart. Also, when a light-sensitive material having the light-sensitive layer coated on a support and a fixing material having the dye fixing layer coated on a support are separately formed, after the light-sensitive material is exposed imagewise to light and uniformly heated, the mobile dye can be transferred on the dye fixing layer by superposing the fixing material on the light-sensitive layer.

Further, there is a method wherein only the light-sensitive layer is exposed imagewise to light and heated uniformly by superposing the dye fixing layer on the light-sensitive layer.

The dye fixing layer can contain, for example, a dye mordant in order to fix the dye. In the present invention, various mordants can be used, and polymer mordants are particularly preferred. In addition to the mordants, the dye fixing layer may contain the bases, base precursors and thermal solvents. In particular, it is particularly preferred to incorporate the bases or base precursors into the dye fixing layer in the cases wherein the light-sensitive layer and the dye fixing layer are formed on different supports.

Preferred polymer mordants used in the present invention can be polymers containing secondary and tertiary amino groups, polymers containing nitrogen-containing heterocyclic moieties, polymers having quaternary cation groups thereof, having a molecular weight of from 5,000 to 200,000, and particularly from 10,000 to 50,000.

For example, vinylpyridine polymers and vinylpyridinium cation polymers as disclosed in U.S. Patents 2,548,564, 2,484,430, 3,148,061 and 3,756,814, polymer mordants capable of cross-linking with gelatin as disclosed in U.S. Patents 3,625,694, 3,859,096 and 4,128,538, and British Patent, 1,277,453, aqueous sol type mordants as disclosed in U.S. Patents 3,958,995, 2,721,852 and 2,798,063 and Japanese Patent Application Nos. 115228/79, 145529/79 and 126027/79, water-insoluble mordants as disclosed in U.S. Patent 3,898,088, reactive mordants capable of forming cobalent bonds with dyes used as disclosed in U.S. Patent 4,168,976 (Japanese Patent Application No. 137333/79), and mordants disclosed in U.S. Patents, 3,709,690, 3,788,855, 3,642,482, 3,488,706, 3,557,066, 3,271,147 and 3,271,148, and Japanese Patent Application Nos. 71332/75, 30328/78, 155528/77, 125/78 and 1024/78, can be illustrated.

In addition, mordants disclosed in U.S. Patents 2,675,316 and 2,882,156 can be used.

The dye fixing layer can have a white reflective layer. For example, a layer of titanium dioxide dispersed in gelatin can be provided on the mordant layer on a transparent support. The layer of titanium dioxide forms

a white opaque layer, by which reflection color images of the transferred color images which can be observed through the transparent support is obtained.

A typical dye fixing material used in the present invention is obtained by mixing the polymer containing ammonium salt groups with gelatin and applying the mixture to a transparent support.

The transfer of dyes from the light-sensitive layer to the dye fixing layer can be carried out using a dye transfer assistant.

In the process in which the dye transfer assistants are supplied from the outside, water and an aqueous solution containing sodium hydroxide, potassium hydroxide or an inorganic alkali metal salt can be used. Further, a solvent having a low boiling point such as methanol, N,N-dimethylformamide, acetone, or diisobutyl ketone, and a mixture of such a solvent having a low boiling point with water or an alkaline aqueous solution can be used. The dye transfer assistant may be applied by wetting the dye fixing layer with the transfer assistant.

When the dye transfer assistant is incorporated into the light-sensitive material or the dye fixing material, it is not necessary to supply the transfer assistant from the outside. In this case, the above-described dye transfer assistant may be incorporated into the material in the form of water of crystallization or microcapsules or as a precursor which releases a solvent at a high temperature.

A more preferred pocess is a process wherein a hydrophilic thermal solvent which is solid at a lower temperature and melts at a higher temperature is incorporated into the light-sensitive material or the dye fixing material. The hydrophilic thermal solvent can be incorporated either into any of the light-sensitive material and the dye fixing material or into both of them. Although the solvent can be incorporated into any of the emulsion layer, the intermediate layer, the protective layer and the dye fixing layer, it is preferred to incorporate it into the dye fixing layer and/or adjacent layers thereto.

Examples of the hydrophilic thermal solvents include ureas, pyridines, amides, sulfonamides, imides, alcohols, oximes and other heterocyclic compounds.

As a heating means to transport dyes, various means which are the same as the heating means for heat-development can be employed.

The "hydrophilic thermal solvent" used as the dye transport assistant is defined as a compound which is solid at room temperature but becomes liquid upon heating and which has a ratio of inorganicity to organicity of greater than 1 and a solubility in water at room temperature of greater than 1. The inorganicity and organicity are the concepts used to predict the properties of chemical compounds. For details of these concepts, see, for example, "Kagaku no Rhoiki (Chemical Domain)", *11*, p. 719 (1957).

The hydrophilic thermal solvents are used to aid in the transport of the hydrophilic dye, so compounds capable of dissolving the hydrophilic dye are generally preferred as such solvents. It is empirically known that preferred solvents for a certain organic compound are those which have a ratio of inorganicity to organicity nearly approaching that of the organic compound. The dye-releasing redox compound used in the present invention has a ratio of inorganicity to organicity of about 1, whereas the hydrophilic dye to be released from this dye-releasing redox compound has a greater value than the dye-releasing compound, preferably 1.5 or more, and more preferably at least 2. Since the hydrophilic thermal solvent used in the present invention preferably transports only the hydrophilic dye, but not the dye-releasing redox compound, it is necessary that the hydrophilic thermal solvent has a ratio of inorganicity to organicity greater than that of the dye donating material. Therefore, the hydrophilic thermal solvent must have a ratio of inorganicity to organicity of at least 1, and the value of at least 2 is preferred.

From a molecular size viewpoint, it is generally preferred that the dye being transported is surrounded by a molecule that will not interfere with the dye transport and which is capable of moving by itself. Therefore, the hydrophilic thermal solvent preferably has a small molecular weight, usually not more than about 200, and a particularly preferred molecular weight is not more than 100.

In order to ensure the extended storage and easy handling of the photographic material, the heating temperature for causing dye transport should be within the range of 60 to 250°C. Therefore, compounds that exhibit the function of the hydrophilic thermal solvent within this temperature range may be properly selected. The hydrophilic thermal solvent must of course be able to aid in the rapid transport of dye upon heating, and in view of the limited heat resistance of the photographic material, the hydrophilic thermal solvent is required to have a melting point of 40 to 250°C, preferably 40 to 200°C, more preferably 40 to 150°C.

The hydrophilic thermal solvent used in the present invention fulfills its function only if it effectively aids in the transport to the dye fixing layer of the hydrophilic due formed by heat development. Therefore, the hydrophilic thermal solvent can be incorporated in various layers: it may be incorporated in the dye fixing layer; it may be incorporated in the light-sensitive layer in the photographic material; it may be incorporated in both the dye fixing layer and the light-sensitive layer; or an independent layer containing the hydrophilic thermal solvent may be incorporated in either the photographic material or a separate dye fixing material having the dye fixing layer. For the purpose of increasing the efficiency of transport to the dye fixing layer, the hydrophilic thermal

solvent is preferably incorporated in the dye fixing layer and/or an adjacent layer.

The hydrophilic thermal solvent is usually dissolved in water before it is dispersed in a binder, but it may be dissolved in alcohols such as methanol and ethanol. The hydrophilic thermal solvent according to the present invention is applied in an amount of generally 5 to 500 wt%, preferably 20 to 200 wt%, more preferably 30 to 150 wt%, of the total coating weight of the photographic material and/or the dye fixing material.

When the dye fixing layer is positioned on top of the photographic material, a protective layer may be further provided as required. Any of the protective layers conventionally used in photographic materials will serve the purpose. However, if the dye fixing layer is incorporated in a dye fixing material separate from the photographic material, it is preferred that the protective layer be also rendered hydrophilic so as not to interfere with the transport of the hydrophilic dye.

In the photographic light-sensitive material and the dye fixing material of the present invention, the photographic emulsion layer and other binder layers may contain inorganic or organic hardeners. It is possible to use chromium salts (chromium alum or chromium acetate), aldehydes (formaldehyde, glyoxal or glutaraldehyde), N-methylol compounds (dimethylolurea or methylol dimethylhydantoin), dioxane derivatives (2,3-dihydroxydioxane), active vinyl compounds (1,3,5-triacryloylhexahydro-s-triazine or 1,3-vinylsulfonyl-2-propanol), active halogen compounds (2,4-dichloro-6-hydroxy-s-triazine), or mucohalogenic acids (mucochloric acid, or mucophenoxychloric acid), which are used individually or as a combustion thereof.

By use of the heat-developable photographic material of the present invention, the development time can be shortened, and in addition, a dye image of good quality having high density and low fog can be obtained. As a further advantage, the heat-developable photographic material of the present invention is very convenient to use because it can be stored for a very long period before effecting heat development and yet has minimum variation in the reproduction of color image.

The image forming process using the photographic material of the present invention having these features can not only be used in photography but also meets the recent demand for conversion from "soft" images to "hard" images. Furthermore, the dye image formed in the photographic material can be fixed in the dye fixing layer, so the image has not only good color reproduction but also improved archival quality. Therefore, the photographic material of the present invention can be conveniently used to meet the demand for extended image storage. For these reasons, the present invention surpasses the conventional photographic techniques and will make a great contribution to the art.

The advantages of the present invention are described in greater detail by reference to the following examples:

Example 1

Gelatin (40 g) and KBr (26 g) were dissolved in water (3,000 ml), and the resulting solution was stirred at 50°C. A solution of silver nitrate (34 g) in water (200 ml) was added to the aqueous solution of gelatin and KBr over a period of 10 minutes. Thereafter, a solution of potassium iodide (3.3 g) in water (100 ml) was added to the mixture over 2 minutes.

The pH of the resulting silver iodobromide emulsion was so adjusted as to precipitate excess salt. After removing the precipitated salt, the supernatant was adjusted to a pH of 0.6. By this procedure, a silver iodobromide emulsion was obtained in a yield of 400 g.

Two dispersons of a dye-releasing redox compound were prepared in the following manner.

Dispersion (A) containing the developing agent of the present invention:

Five grams of a magenta dye donating material (a) followed below, 0.01 g of a developing agent,(i-24), 0.5 g of a sodium di-1,2-(2-ethylhexyloxycarbonyl) ethylsulphonate and 15 g of tri-cresyl phosphate (TCP) were dissolved in ethyl acetate (30 ml) and heated at about 60°C to form a uniform solution. This solution was mixed under stirring with 100g of a 10% solution of lime treated gelatin. The mixture was homogenized in a homogenizer for 10 minutes at 10,000 rpm to prepare dispersion (A) of dye-releasing redox compound (a).

Dispersion (B) containing no developing agent:

The procedure of the preparation of dispersion (A) was repeated except that developing agent (i-24) was not used.

(a)

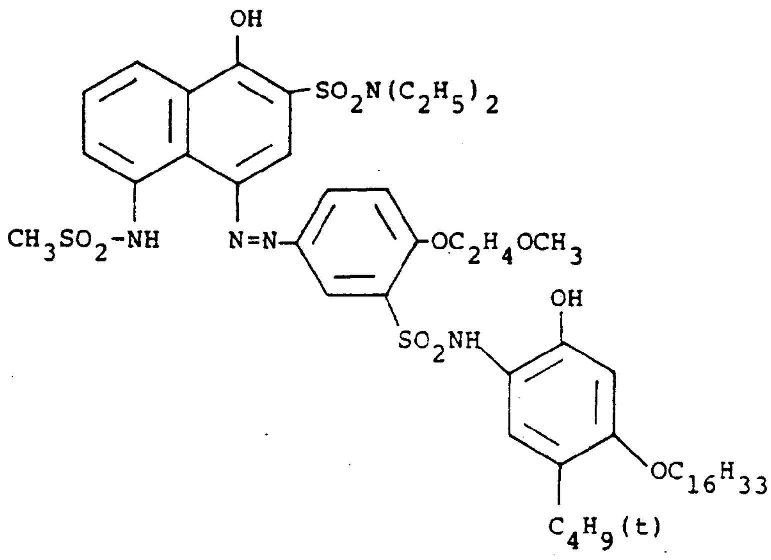

Photographic materials A* and B* were prepared in the following manner.

Photographic material A*:

The components listed below were mixed to form a solution.

| | |
|---|---|
| (a) Silver iodobromide emulsion (prepared as above) | 25 g |
| (b) Dispersion (A) of dye-releasing redox compound (a) containing developing agent (i-24) | 33 g |
| (c) 5% aqueous solution of the following compound | 5 ml |

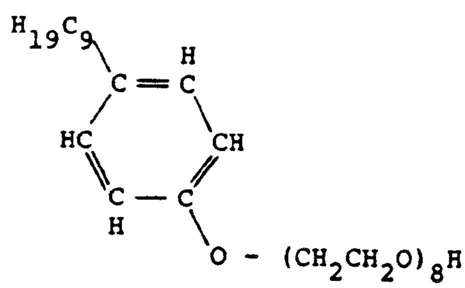

| | |
|---|---|
| (d) 10% ethanol solution of guanidine trichloroacetic acid | 12 ml |
| (e) 10% aqueous solution of dimethylsulfamide | 4 ml |
| (f) Water | 8 ml |

The solution was coated onto a polyethylene terephthalate film to give a wet thickness of 30 μm and subsequently dried. Then, the following components were mixed.

| | |
|---|---|
| (i) 10% aqueous solution of gelatin | 35 g |
| (ii) 10% ethanol solution of guanidine trichloroacetic acid | 5 ml |
| (iii) 1% aqueous solution of sodium sulfonate of 2-ethyl-hexyl succinate | 4 ml |
| (iv) Water | 56 ml |

The resulting solution was applied to the previously formed light-sensitive layer to provide a protective layer in a wet thickness of 25 μm and subsequently dried to make photographic material A*.

Photographic material B* was prepared by repeating the above procedure except that Dispersion (A) of dye donating material (a) containing developing agent (i-24) was replaced by Dispersion (B) of dye-releasing redox compound (a) containing no developing agent.

The two photographic materials were subjected to imagewise exposure under a tungsten halogen lamp (2000 lux) for 10 seconds, and heated uniformly for 15 seconds on a heated block at 130°C.

Two samples of a dye fixing material having a dye fixing agent were prepared in the following manner.

Ten grams of poly(methyl acrylate-N,N,N-trimethyl-N-vinylbenzylammonium chloride) (the ratio of methyl acrylate to vinylbenzylammonium chloride being 1:1) was dissolved in 200 ml of water, and the resulting solution was uniformly mixed with 100 g of 10% lime treated gelatin. The mixture was uniformly applied to a polyethylene-laminated paper support (containing dispersed particles of titanium dioxide) to give a wet thickness of 90 μm.

After immersion in water, one sample of the dye fixing material was superimposed on heated photographic material A* so that the layer of the dye fixing agent was in contact with the photographic material. The other sample of the dye fixing material was superimposed on heated photographic material B* in the same manner. Each assembly was heated for 6 seconds on a heated block (80°C). The two samples of dye fixing material were peeled from the respective photographic materials, leaving a negative magenta color image on each sample of the dye fixing material. The maximum, and minimum densities of each negative image were measured with a Macbeth reflection densitometer (RD-519). The results are shown below.

| Sample No. | $D_{max}$ | $D_{min}$ |
|---|---|---|
| A* (sample of the present invention) | 2.08 | 0.20 |
| B* (comparative sample) | 1.30 | 0.16 |

The above results show that the developing agent of the present invention is highly effective in obtaining a high image density by heating for a short period.

Example 2

Dispersions (C), (D), (E), (F), (G) and (H) of dye-releasing redox compound (a) were prepared as in Example 1 except that developing agent (i-24) was replaced by the compounds shown in the following table. Using these dispersions, photographic materials (C*, D*, E*, F*, G* and H*) were prepared as in Example 1 and they were processed as in Example 1. The results are shown in the following table.

| Dispersion of dye-releasing redox compound | Developing agent | $D_{max}$ | $D_{min}$ |
|---|---|---|---|
| B (comparative dispersion) | None | 1.30 | 0.16 |
| C (dispersion of the present invention) | i-13 | 2.00 | 0.19 |
| D (dispersion of the present invention) | i-14 | 1.95 | 0.18 |
| E (dispersion of the present invention) | i-17 | 1.98 | 0.18 |
| F (dispersion of the present invention) | i-25 | 2.10 | 0.21 |
| G (dispersion of the present invention) | i-40 | 1.95 | 0.18 |
| H (dispersion of the present invention) | i-41 | 1.80 | 0.17 |

The above results show that by use of the heat-developable photographic materials of the present invention having developing agents, high image densities can be obtained by heating for a short period.

Example 3

Dispersion containing a developing agent (i-24) and those containing no developing agent were prepared as in Example 1 except that dye-releasing redox compound material (a) was replaced by the compounds listed

below.

| | | |
|---|---|---|
| Dye-releasing redox compound (b) | 5 g | Dispersion (I) |
| Dye-releasing redox compound (c) | 7.5 g | Dispersion (J) |
| Dye-releasing redox compound (d) | 5 g | Dispersion (K) |

Using these dispersions, photographic materials were prepared as in Example 1 and were then processed as in Example 1. The results are shown in the following table.

- Dye-releasing redox compound (b)

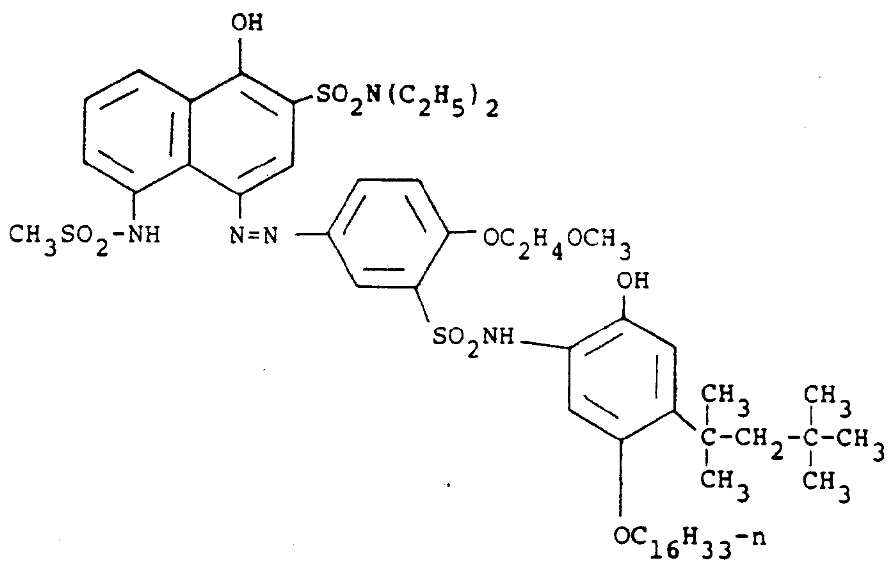

- Dye-releasing red compound (c)

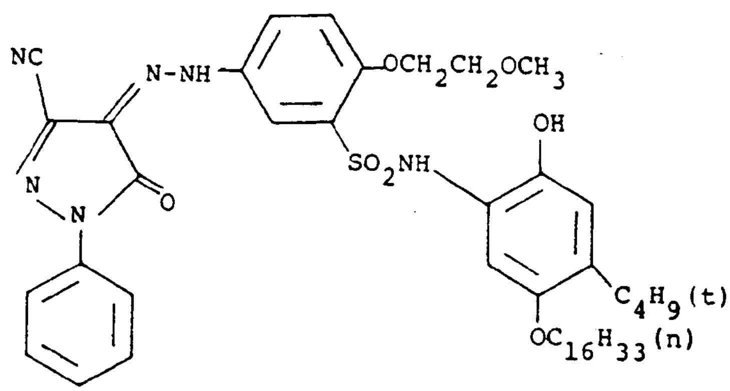

- Dye-releasing redox compound (d)

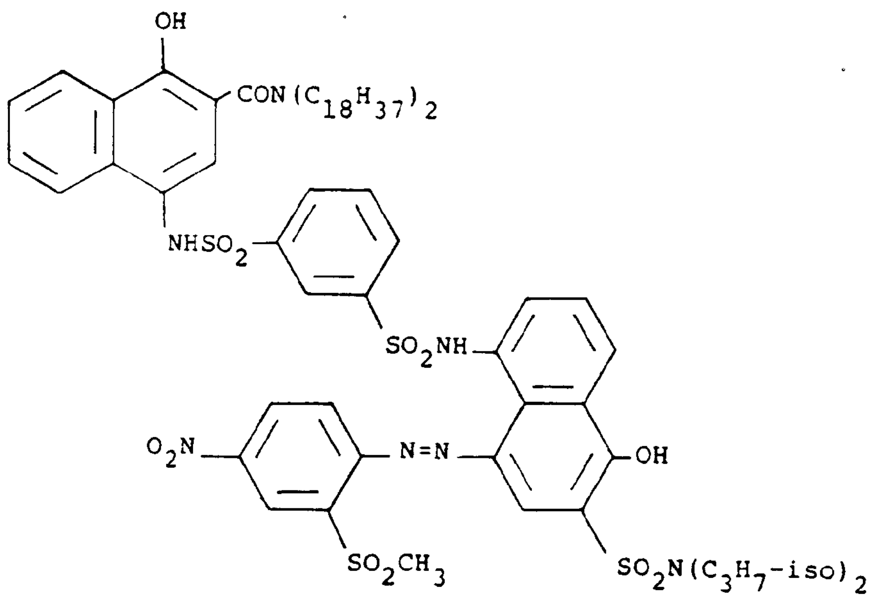

| Dispersion of dye-releasing redox compound | Developing agent (I-24) | $D_{max}$ | $D_{min}$ |
|---|---|---|---|
| (I) magenta | Present (sample of the present invention) | 2.13 | 0.18 |
| | Absent (comparative sample) | 1.33 | 0.12 |
| (J) Yellow | Present (sample of the present invention) | 2.08 | 0.18 |
| | Absent (comparative sample) | 1.28 | 0.11 |
| (K) cyan | Present (sample of the present invention) | 2.16 | 0.19 |
| | Absent (comparative sample) | 1.40 | 0.13 |

The above results show that the photographic materials of the present invention provide high image densities by heating for a short period.

Example 4

This example shows the effectiveness of using an organic silver salt as an oxidizing agent.

Preparation of an emulsion of silver salt of benzotriazole:

Gelatin (28 g) and benzotriazole (13.2 g) were dissolved in water (3,000 ml) and the resulting solution was stirred at 40°C. To the stirred solution, a solution of silver nitrate (17 g) in water (100 ml) was added over 2 minutes.

The pH of the resulting emulsion of silver salt of benzotriazole was so adjusted as to precipitate excess salt. The supernatant was adjusted to a PH of 6.0 to obtain an emulsion of silver salt of benzotriazole in a yield of 400 g.

Using this emulsion, a photographic material was prepared in the following manner.

The components listed below were mixed to form a solution.

| | |
|---|---|
| (a) emulsion of silver salt of benzotriazole | 10 g |
| (b) silver iodobromide emulsion | 20 g |
| (c) Dispersion (a) of dye-releasing redox compound (a) containing developing agent (i-24) | 33 g |
| (d) 5% aqueous solution of the following compound | 5 ml |

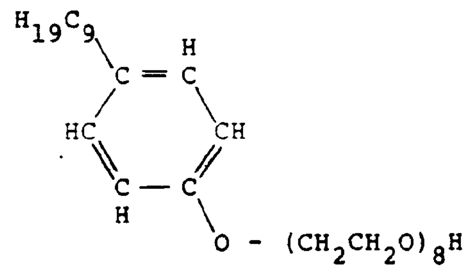

| | |
|---|---|
| (e) 10% ethanol solution of guanidine trichloroacetic acid | 12.5 ml |
| (f) 10% aqueous solution of dimethylsulfamide | 4 ml |
| (g) Water | 7.5 ml |

The solution was coated onto a polyethylene terephthalate film to give a wet thickness of 30 μm and subsequently dried. On the dried light-sensitive layer, a protective layer was formed as in the preparation of photographic material A*. The resulting product was referred to as photographic material A*∗.

Photographic material B*∗ was prepared as above except that Dispersion (A) of dye-releasing redox compound (a) containing developing agent (I-24) was replaced by Dispersion (B) of dye-releasing redox compound (a) containing no developing agent.

The two photographic materials were processed as in Example 1. The results are shown in the following table.

| Sample No. | $D_{max}$ | $D_{min}$ |
|---|---|---|
| A** (sample of the present invention) | 2.11 | 0.21 |
| B** (comparative sample) | 1.32 | 0.17 |

The above results show that the photographic material of the present invention is effective in providing a high value of maximum density by heating for a short period.

Example 5

Two samples each of photographic materials A* and B* (Example 1) and C* to H* (Example 2) were prepared. One group of the eight samples was exposed, heated and transferred as in Example 1. The other group was likewise processed after they were stored for 2 days in a constant temperature chamber (50°C). The reflection density for green light of the image formed on each sample was measured. The results are shown in the following table.

| Sample No. | Developing agent | Fresh | | After storage 50°C × 2 days | |
|---|---|---|---|---|---|
| | | $D_{max}$ | $D_{min}$ | $D_{max}$ | $D_{min}$ |
| A (sample of the present invention) | i-24 | 2.08 | 0.20 | 2.11 | 0.60 |
| B (comparative sample) | None | 1.30 | 0.16 | 1.90 | 1.40 |
| C (sample of the present invention) | i-13 | 2.00 | 0.19 | 2.03 | 0.25 |
| D (sample of the present invention) | i-14 | 1.95 | 0.18 | 2.00 | 0.24 |
| E (sample of the present invention) | i-17 | 1.98 | 0.18 | 2.01 | 0.35 |
| F (sample of the present invention) | i-25 | 2.10 | 0.21 | 2.13 | 0.65 |
| G (sample of the present invention) | i-40 | 1.95 | 0.18 | 2.00 | 0.28 |
| H (sample of the present invention) | i-41 | 1.80 | 0.17 | 1.90 | 0.25 |

The above data show that the photographic materials of the present invention containing developing agents have improved stability and experience only a small increase in minimum density during storage.

Example 6

This example shows the use of a dye fixing material adapted to image formation by a complete dry process.

In this example, photographic materials A* and B* prepared as in Example 1 were used. Two samples of a dye fixing material having a due fixing agent were prepared in the following manner.

Ten grams of poly(methyl acrylate-N,N,N-trimethyl-N-vinylbenzylammonium chloride) (the ratio of methyl acrylate to vinylbenzylammonium chloride being 1:1) was dissolved in 200 ml of water, and the resulting solution was mixed uniformly with 100 g of 10% lime treated gelatin. The mixture was uniformly applied to a polyethylene terephthalate film (120 μm thick and containing dispersed particles of titanium dioxide) to give a wet thickness of 90 μm.

Onto the resulting layer of dye fixing agent, Solution A having the following components (a) to (f) was uniformly applied to give a wet thickness of 60 μm.

| | |
|---|---|
| (a) urea | 2 g |
| (b) N-methylurea | 2 g |
| (c) water | 10 ml |
| (d) 10 wt% aqueous solution of polyvinyl alcohol (98% saponification) | 12 g |
| (e) compound of the following structure | 100 mg |

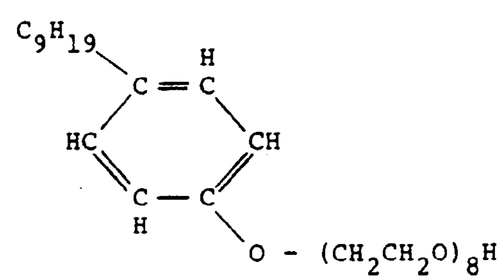

| | |
|---|---|
| (f) 5% aqueous solution of sodium dodecylbenzenesulfonate | 0.5 ml |

The resulting web was dried to form a dye fixing material.

Photographic materials A* and B* were subjected to imagewise exposure under a tungsten halogen lamp (2000 lux) for 10 seconds, and heated for 15 seconds on a heated block at 130°C. One sample of the dye fixing material was superimposed on heated photographic material A* so that the layer of the dye fixing agent was in contact with the photographic material. The other sample of the dye fixing material was superimposed on heated photographic material B* in the same manner. Each assembly was heated for 20 seconds on a heated block (140°C). The two samples of dye fixing material were peeled from the respective photographic materials, leaving a negative magenta color image on each sample of the dye fixing material. The maximum and minimum densities of each negative image were measured with a Macbeth reflection densitometer (RD-519). The results are shown below.

| Sample No. | $D_{max}$ | $D_{min}$ |
|---|---|---|
| A* (sample of the present invention) | 2.10 | 0.48 |
| B* (comparative sample) | 1.25 | 0.34 |

The above data show that the heat-developable photographic material of the present invention provides as good results as obtained in Example 1 even if it is treated by a complete dry process.

## Claims

1. A heat-developable photographic material, which comprises a support having provided thereon a light-sensitive silver halide, a hydrophilic binder, a dye-releasing redox compound which is capable of reducing the light-sensitive silver halide and which upon heating reacts with the light-sensitive silver halide to release a hydrophilic dye and a developing agent characterized in that the developing agent is represented by the following formula i

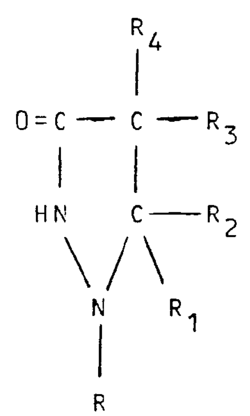

wherein
R represents an unsubstituted or substituted aryl group and
$R_1$, $R_2$, $R_3$ and $R_4$ which may be the same or different represent a hydrogen atom, an unsubstituted or substituted alkyl group, an unsubstituted or substituted aryl group, a carboxy group or an alkoxycarbonyl group with the exclusion of 1-phenyl-3-pyrazolidone and 1-phenyl-4-methyl-4-hydroxymethyl-3-pyrazolidone.

2. The material as claimed in claim 1, wherein the aryl group represented by R has 6 to 22 carbon atoms.

3. The material as claimed in claim 1, wherein the aryl group represented by R is substituted by at least one substituent selected from the group consisting of a halogen atom, an amino group, an alkoxy group, an aryloxy group, a hydroxy group, an aryl group, a carbonamide group, a sulfonamide group, an alkanoyloxy group, a benzoyloxy group, an ureido group, a carbamate group, a carbamoxyloxy group, a carbonate group, a carboxy group and an alkyl group.

4. The material as claimed in claim 1, wherein the alkyl group, the argyl group, the carboxy group and the alkoxycarbonyl group represented by $R_1$, $R_2$, $R_3$ and $R_4$ have 1 to 10 carbon atoms, 6 to 22 carbon atoms, 1 to 22 carbon atoms and 2 to 22 carbon atoms, respectively.

5. The material as claimed in claim 1, wherein the alkyl group represented by $R_1$, $R_2$, $R_3$ and $R_4$ is substituted by at least one substituent selected from the group consisting of a hydroxy group, an amino group, a carbonyloxy group and an alkoxycarbonyl group.

6. The material as claimed in claim 1, wherein the aryl group represented by $R_1$, $R_2$, $R_3$ and $R_4$ is substituted by at least one substituent selected from the group consisting of a halogen atom, an alkyl group, a hydroxy group and an alkoxy group.

7. The material as claimed in claim 1, wherein the developing agent represented by the general formula i is incorporated in an amount of 0.0005 mol to 20 mols per mol of silver.

8. The material as claimed in claim 1, wherein the heat-developable photographic material contains an organic silver salt.

9. The material as claimed in claim 1, wherein the support has further provided thereon a dye-fixing layer.

10. The material as claimed in claim 1, wherein the photographic material is superposed by a fixing material comprising a support having provided thereon a dye-fixing layer.

11. The material as claimed in claims 9 and 10, wherein the dye-fixing layer contains a dye mordant.

12. The material as claimed in claim 1, wherein the dye-providing substance capable of reducing the light-sensitive silver halide and releasing a hydrophilic dye is represented by the following general formula I:

$$RaSO_2\text{-}D \qquad (I)$$

wherein Ra represents a reductive group capable of being oxidized with the silver halide, and D represents an image-forming dye portion containing a hydrophilic dye.

13. The material as claimed in claim 1, wherein the light-sensitive material further contains a dye-releasing activator.

14. The material as claimed in claim 13, wherein the dye-releasing activator is a base or a base precursor.

15. The material as claimed in claim 8, wherein the organic silver salt is a compound selected from the group consisting of a silver salt of a carboxylic acid derivative and a nitrogen-containing hetero ring compound.

16. The material as claimed in claim 1, wherein the binder is gelatin or a gelatin derivative.

17. The material as claimed in claim 1, wherein the light-sensitive silver halide is selected from the group consisting of silver chloride, silver chlorobromide, silver chloroiodide, silver bromide, silver iodobromide, silver chloroiodobromide and silver iodide.

## Patentansprüche

1. Wärmeentwickelbares photographisches Material, umfassend einen Träger, auf dem ein lichtempfindliches Silberhalogenid, ein hydrophiles Bindemittel, eine Farbstoff freisetzende Redoxverbindung, die fähig ist, das lithtempfindliche Silberhalogenid zu reduzieren und die beim Erwärmen mit dem lichtempfindlichen Silberhalogenid reagiert, um einen hydrophilen Farbstoff freizusetzen, und ein Entwicklermittel vorgesehen sind, dadurch gekennzeichnet, daß das Entwicklermittel der folgenden allgemeinen Formel i entspricht:

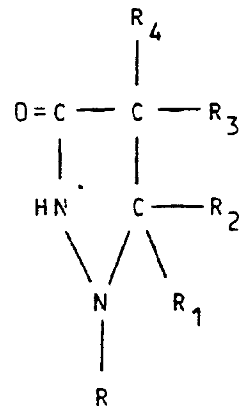

worin bedeuten:

R eine unsubstituierte oder substituierte Arylgruppe und

$R_1$, $R_2$, $R_3$ und $R_4$, die gleich oder verschieden sein können, ein Wasserstoffatom, eine unsubstituierte oder substituierte Alkylgruppe, unsubstituierte oder substituierte Arylgruppe, Carboxygruppe oder Alkoxycarbonylgruppe,

ausgenommen 1-Phenyl-3-pyrazolidon und 1-Phenyl-4-methyl-4-hydroxymethyl-3-pyrazolidon.

**2.** Material nach Anspruch 1, wobei die durch R dargestellte Arylgruppe 6 bis 22 Kohlenstoffatome besitzt.

**3.** Material nach Anspruch 1, wobei die durch R dargestellte Arylgruppe durch mindestens einen Substituenten substituiert ist, gewält aus der Gruppe, bestehend aus einem Halogenatom, einer Aminogruppe, Alkoxygruppe, Aryloxygruppe, Hydroxygruppe, Arylgruppe, Carbonamidgruppe, Sulfonamidgruppe, Alkanoyloxygruppe, Benzoyloxygruppe, Ureidogruppe, Carbamatgruppe, Carbamoyloxygruppe, Carbonatgruppe, Carboxygruppe und Alkylgruppe.

**4.** Material nach Anspruch 1, wobei die Alkylgruppe, Arylgruppe, Carboxygruppe und Alkoxycarbonylgruppe, wie durch $R_1$, $R_2$, $R_3$ und $R_4$ angegeben, 1 bis 10 Kohlenstoffatome, 6 bis 22 Kohlenstoffatome, 1 bis 22 Kohlenstoffatome bzw. 2 bis 22 Kohlenstoffatome besitzen.

**5.** Material nach Anspruch 1, wobei die durch $R_1$, $R_2$, $R_3$ und $R_4$ dargestellte Alkylgruppe durch mindestens einen Substituenten substituiert ist, gewählt aus der Gruppe, bestehend aus einer Hydroxygruppe, Aminogruppe, Carbonyloxygruppe und Alkoxycarbonylgruppe.

**6.** Material nach Anspruch 1, wobei die durch $R_1$, $R_2$, $R_3$ und $R_4$ dargestellte Arylgruppe durch mindestens einen Substituenten substituiert ist, gewählt aus der Gruppe, bestehend aus einem Halogenatom, einer Alkylgruppe, Hydroxygruppe und Alkoxygruppe.

**7.** Material nach Anspruch 1, wobei das durch die allgemeine Formel i angegebene Entwicklermittel in einer Menge von 0,0005 Mol bis 20 Mol pro Mol Silber enthalten ist.

**8.** Material nach Anspruch 1, wobei das wärmeentwickelbare photographische Material ein organisches Silbersalz enthält.

**9.** Material nach Anspruch 1, wobei auf dem Träger weiterhin eine Farbstoff fixierende Schicht vorgesehen ist.

**10.** Material nach Anspruch 1, wobei das photographische Material von einem Fixiermaterial, umfassend einen Träger mit einer darauf vorgesehenen Farbstoff fixierenden Schicht, überschichtet ist.

**11.** Material nach den Ansprüchen 9 und 10, wobei die Farbstoff fixierende Schicht ein Farbstoff-Beizmittel enthält.

**12.** Material nach Anspruch 1, wobei die Farbstoff vorsehende Substanz, die fähig ist, das Lichtempfindliche Silberhalogenid zu reduzieren und einen hydrophilen Farbstoff freizusetzen, der folgen allgemeinen For-

mel I entspricht:

$$Ra\text{-}SO_2\text{-}D \qquad (I)$$

worin Ra eine reduzierende Gruppe, die in der Lage ist, von dem Silberhalogenid oxidiert zu werden, und D einen bilderzeugenden Farbstoffteil, der einen hydrophilen Farbstoff enthält, bedeuten.

**13.** Material nach Anspruch 1, wobei das lichtempfindliche Material weiterhin einen Farbstoffreisetzungs-Aktivator enthält.

**14.** Material nach Anspruch 13, wobei der Farbstoffreisetzungs-Aktivator eine Base oder ein Basenvorläufer ist.

**15.** Material nach Anspruch 8, wobei das organische Silbersalz eine Verbindung ist, gewählt aus der Gruppe, bestehend aus einem Silbersalz eines Carbonsäurederivatives und einer Stickstoff enthaltenden Heteroringverbindung.

**16.** Material nach Anspruch 1, wobei das Bindemittel Gelatine oder eine Gelatinederivat ist.

**17.** Material nach Anspruch 1, wobei das lichtempfindliche Silberhalogenid gewählt wird aus der Gruppe, bestehend aus Silberchlorid, Silberchlorbromid, Silberchlorjodid, Silberbromid, Silberjodbromid, Silberchlorjodbromid und Silberjodid.

## Revendications

**1.** Matériau photographique développable à chaud, comprenant un support sur lequel sont placés un halogénure d'argent photosensible, un liant hydrophile, un componé redox libérant un colorant qui est capable de réduire l'halogénure d'argent photosensible et qui réagit au chauffage avec l'halogénure d'argent photosensible pour libérer un colorant hydrophile, et un développateur, caractérisé en ce que le développateur est représenté par la formule suivante :

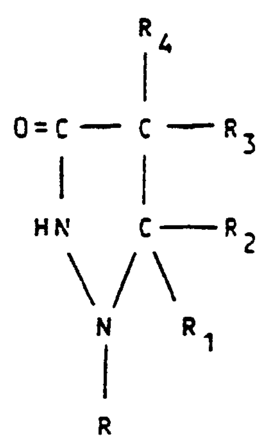

dans laquelle R représente un groupe aryle insubstitué ou substitué, et $R_1$, $R_2$, $R_3$ et $R_4$ qui peuvent être identiques ou différents, représentent un atome d'hydrogène, un groupe alkyle insubstitué ou substitué, un groupe aryle insubstitué ou substitué, un groupe carboxy, ou un groupe alcoxycarbonyl, à l'exclusion de la 1-phényl-3-pyrazolidone et de la 1-phényl-4-méthyl -4-hydroxyméthyl-3-pyrazolidone.

**2.** Le matériau suivant la revendication 1, dans lequel le groupe aryle représenté par R a 6 à 22 atomes de carbone.

**3.** Le matériau suivant la revendication 1, dans lequel le groupe aryle représenté par R est substitué par au moins un substituant choisi dans le groupe constitué par un atome d'halogène, un groupe amino, un groupe alcoxy, un groupe aryloxy, un groupe hydroxy, un groupe aryle, un groupe carbonamide, un groupe sulfonamide, un groupe alcanoyloxy, un groupe benzoyloxy, un groupe uréido, un groupe carbamate, un groupe carbamyloxy, un groupe carbonate, un groupe carboxy, et un groupe alkyle.

**4.** Le matériau suivant la revendication 1, dans lequel le groupe alkyle, le groupe aryle, le groupe carboxy, et le groupe alcoxycarbonyle représentés par $R_1$, $R_2$, $R_3$ et $R_4$ ont de 1 à 10 atomes de carbone, de 6 à 22 atomes de carbone, de 1 à 22 atomes de carbone, et de 2 à 22 atomes de carbone, respectivement.

**5.** Le matériau suivant la revendication 1, dans lequel le groupe alkyle représenté par $R_1$, $R_2$, $R_3$ et $R_4$ est substitué par au moins un substituant choisi dans le groupe constitué par un groupe hydroxy, un groupe amino, un groupe carbonyloxy, et un groupe alcoxycarbonyle.

**6.** Le matériau suivant la revendication 1, dans lequel le groupe aryle represente par $R_1$, $R_2$, $R_3$ et $R_4$ est substitué par au moins un substituant choisi dans le groupe constitué par un atome d'halogène, un groupe alkyle, un groupe hydroxy et un groupe alcoxy.

**7.** Le matériau suivant la revendication 1, dans lequel le développateur représenté par la formule générale i est incorporé en une quantité de 0,0005 à 20 moles par mole d'argent.

**8.** Le matériau suivant la revendication 1, dans lequel le matériau photographique développable à chaud contient un sel d'argent organique.

**9.** Le matériau suivant la revendication 1, dans lequel le support porte en outre une couche fixatrice de colorant.

**10.** Le matériau suivant la revendication 1, dans lequel un matériau fixateur comprenant un support sur lequel est placée une couche fixatrice de colorant, est superposé sur le matériau photographique.

**11.** Le matériau selon les revendications 9 et 10, caractérisé en ce que la couche fixatrice de colorant contient un mordant colorant.

**12.** Le matériau suivant la revendication 1, dans lequel la substance procurant un colorant capable de réduire l'halogénure d'argent photosensible et de libérer un colorant hydrophile est représentée par la formule génerale I suivante:

$$Ra\text{-}SO_2\text{-}D \qquad (I)$$

dans laquelle Ra représente un groupe réducteur capable d'être oxydé par l'halogénure d'argent, et D représente une partie colorant formateur d'image contenant un colorant hydrophile.

**13.** Le matériau suivant la revendication 1, dans lequel le matériau photosensible contient en outre un activateur de libération du colorant.

**14.** Le matériau suivant la revendication 13, dans lequel l'activateur de libération du colorant est une base ou un précurseur de base.

**15.** Le matériau suivant la revendication 8, dans lequel le sel d'argent organique est un comosé choisi dans le groupe constitué par un sel d'argent de dérivé d'acide carboxylique et un composé hétérocyclique contenant de l'azote.

**16.** Le matériau suivant la revendication 1, dans lequel le liant est la gélatine ou un dérivé de gélatine.

**17.** Le matériau suivant la revendication 1, dans lequel l'halogénure d'argent photosensible est choisi dans le groupe constitué par le chlorure d'argent, le chlorobromure d'argent, le chloroiodure d'argent, le bromure d'argent, le iodobromure d'argent, le chloroiodobromure d'argent, et l'iodure d'argent.